(19) Europäisches Patentamt
European Patent Office
Office européen des brevets

(11) **EP 4 330 835 B1**

(12) **EUROPEAN PATENT SPECIFICATION**

(45) Date of publication and mention
of the grant of the patent:
**17.12.2025 Bulletin 2025/51**

(21) Application number: **22866795.2**

(22) Date of filing: **13.09.2022**

(51) International Patent Classification (IPC):
**G06T 11/00** (2006.01)    **G06F 18/00** (2023.01)
**G06N 3/04** (2023.01)    **G06T 5/00** (2024.01)
**G16H 30/20** (2018.01)    **G06N 3/08** (2023.01)
**G06T 5/50** (2006.01)    **G06T 5/70** (2024.01)

(52) Cooperative Patent Classification (CPC):
**G06F 18/214; G06N 3/0464; G06N 3/09;
G06T 5/60; G06T 5/70; G06T 11/008; G06V 10/82;
G16H 30/20; G16H 30/40; G16H 50/20;**
G06T 2207/10081; G06T 2207/20081;
G06T 2207/20084; G06T 2211/408; G06T 2211/441;
(Cont.)

(86) International application number:
**PCT/CN2022/118439**

(87) International publication number:
**WO 2023/036334 (16.03.2023 Gazette 2023/11)**

(54) **METHODS AND SYSTEMS FOR IMAGE PROCESSING**

VERFAHREN UND SYSTEME ZUR BILDVERARBEITUNG

PROCÉDÉS ET SYSTÈMES DE TRAITEMENT D'IMAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.09.2021   CN 202111070115**

(43) Date of publication of application:
**06.03.2024 Bulletin 2024/10**

(73) Proprietor: **Shanghai United Imaging Healthcare
Co., Ltd.
Shanghai 201807 (CN)**

(72) Inventors:
• **YANG, Meili
Shanghai 201807 (CN)**
• **FU, Jianwei
Shanghai 201807 (CN)**

(74) Representative: **Wang, Bo
Panovision IP
Ebersberger Straße 3
85570 Markt Schwaben (DE)**

(56) References cited:
CN-A- 103 649 990    CN-A- 108 010 098
CN-A- 110 958 856    CN-A- 111 145 281
CN-A- 113 298 905    CN-A- 113 706 419
US-A1- 2021 104 023    US-A1- 2021 104 023

• ZHIPENG LI ET AL: "An Improved Iterative Neural
Network for High-Quality Image-Domain Material
Decomposition in Dual-Energy CT", ARXIV.ORG,
CORNELL UNIVERSITY LIBRARY, 201 OLIN
LIBRARY CORNELL UNIVERSITY ITHACA, NY
14853, 2 December 2020 (2020-12-02),
XP081828982
• LI ZHIPENG ET AL: "Image-Domain Material
Decomposition Using an Iterative Neural
Network for Dual-Energy CT", 2020 IEEE 17TH
INTERNATIONAL SYMPOSIUM ON BIOMEDICAL
IMAGING (ISBI), IEEE, 3 April 2020 (2020-04-03),
pages 651 - 655, XP033773707, DOI: 10.1109/
ISBI45749.2020.9098590

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LI MOHAN ET AL: "A study on noise reduction for dual-energy CT material decomposition with autoencoder", vol. 3, no. 3, 1 September 2019 (2019-09-01), Singapore, XP055954687, ISSN: 2509-9930, Retrieved from the Internet <URL:http://link.springer.com/article/10.1007/s41605-019-0122-2/fulltext.html> DOI: 10.1007/s41605-019-0122-2**

(52) Cooperative Patent Classification (CPC): (Cont.)
G06V 2201/03

**Description**

**CROSS-REFERENCE TO RELATED APPLICATIONS**

This application claims priority to Chinese Patent Application No. 202111070115.4, filed on September 13, 2021.

**TECHNICAL FIELD**

The present disclosure relates to the field of image processing, in particular, to image processing method and system thereof.

**BACKGROUND**

Computed tomography (CT) has important application values in the hepatic fibrous fibrosis, breast tumor diagnosis, spinal compression fracture diagnosis, and renal stones analysis. Usually, two attenuation values collected on two different energy spectra may be used to determine the photoelectric and Compton contribution values, which include the mass attenuation coefficient of the material, so as to identify the unknown material and realize material decomposition through its photoelectric and Compton contribution values. However, the existing CT imaging method is facing some inconvenience in actual application. For example, the signal-to-noise ratio of a material density image decomposed using material decomposition or least square decomposition of one or more CT images directly through matrix inversion may be seriously degraded.

Therefore, it is desirable to provide image processing methods to obtain a material density image with a high signal-to-noise ratio and a low noise.

XP81828982A (ZHIPENG LI ET AL: "An Improved Iterative Neural Network for High-Quality Image-Domain Material Decomposition in Dual-Energy CT", ARXIV.ORG, CORNELL UNIVERSITY LIBRARY, 201 OLIN LIBRARY CORNELL UNIVERSITY ITHACA, NY 14853, 2 December 2020 (2020-12-02)) relates to new INN architecture, distinct cross-material BCD-Net, for DECT material decomposition. XP33773707A (LI ZHIPENG ET AL: "Image-Domain Material Decomposition Using an Iterative Neural Network for Dual-Energy CT", 2020 IEEE 17TH INTERNATIONAL SYMPOSIUM ON BIOMEDICAL IMAGING (ISBI), IEEE, 3 April 2020 (2020-04-03), pages 651-655) relates to a new INN architecture for DECT material decomposition by replacing a model-based image reconstruction module of BCD-Net with a model-based image decomposition (MBID) module.

**SUMMARY**

The invention is set out in the appended set of claims.

**BRIEF DESCRIPTION OF THE DRAWINGS**

The present disclosure will be further described in the form of exemplary embodiments, which will be described in detail by the accompanying drawings. These embodiments are not restrictive. In these embodiments, the same number represents the same structure, wherein:

> FIG. 1 is an exemplary schematic diagram illustrating an exemplary application scenario of an image processing system according to some embodiments of the present disclosure;
> FIG. 2 is a schematic diagram of hardware and/or software of an exemplary computing device according to some embodiments of the present disclosure;
> FIG. 3 is a schematic diagram of an exemplary image processing system according to some embodiments of the present disclosure;
> FIG. 4 is a flowchart of an exemplary image processing process according to some embodiments of the present disclosure;
> FIG. 5 is a flowchart of an iterative calculation process of an exemplary image processing model according to some embodiments of the present disclosure; and
> FIG. 6 is a flowchart of an exemplary image processing model determination process according to some embodiments of the present disclosure.

**DETAILED DESCRIPTION**

In order to more clearly explain the technical scheme of the embodiments of the present disclosure, the following will briefly introduce the drawings that need to be used in the description of the embodiments. Obviously, the drawings in the following description are only some examples or embodiments of the present disclosure. For those skilled in the art, the present disclosure can also be applied to other similar scenarios according to these drawings without creative work. Unless it is obvious from the language environment or otherwise stated, the same label in the figure represents the same structure or operation.

It should be understood that the "system", "device", "unit" and/or "module" used herein is a method for distinguishing different components, elements, components, parts or assemblies at different levels. However, if other words serve the same purpose, they may be replaced by other expressions.

As shown in the description and claims, the words "one", and/or "this" do not specifically refer to the singular, but may also include the plural, unless the context clearly indicates exceptions. Generally speaking, the terms "include" and "comprise" only indicate that the steps and elements that have been clearly identified are included, and these steps and elements do not constitute an exclusive list. Methods or equipment may also contain other steps or elements.

The terms "including", "having" and any deformation thereof referred to in the present disclosure are intended to cover non exclusive inclusion; For example, a process, method, system, product or device that includes a series of steps or modules (units) is not limited to the listed steps or units, but may also include steps or units that are not listed, or may also include other steps or units inherent to these processes, methods, products or devices. The "a plurality of" involved in the present disclosure refers to more than or equal to two. "And/or" describes the association relationship of the associated object, indicating that there may be three relationships. For example, "A and/or B" can indicate that there are three situations: A alone, A and B at the same time, and B alone. The terms "first", "second", "third" and "fourth" involved in the present disclosure only distinguish similar objects and do not represent a specific ranking of objects.

A flowchart is used in the present disclosure to explain the operation performed by the system according to the embodiment of the present disclosure. It should be understood that the preceding or subsequent operations are not necessarily performed accurately in sequence. Instead, you can process the steps in reverse order or simultaneously. At the same time, you can add other operations to these procedures, or remove one or more operations from these procedures.

Computed Tomography (CT) is widely used in clinical diagnosis. Photon counting CT can be used to count photons in different energy intervals by setting several thresholds, so as to obtain projection of X-rays with different energy intervals after passing through an object, and reconstruct an energy spectrum CT image, thereby obtaining scanning data of a plurality of different energy segments.

Decomposition of various material densities may be realized based on data of a plurality of energy segments. However, because a matrix including material density decomposition coefficients has one or more relatively large condition numbers, a signal-to-noise ratio of a material density image obtained using direct matrix inversion material decomposition or least square decomposition may be seriously degraded. In some embodiments, at least one of material noise reduction processing or artifact removal processing may be performed on one or more material density images obtained by the direct matrix inversion material decomposition and/or the least square decomposition using iterative material decomposition or a convolution network model for noise reduction. However, the iterative material decomposition is slow, which is difficult to meet clinical needs, and it is difficult to find a good penalty function to improve image quality. Directly using the convolution network model for the material noise reduction processing and/or the artifact removal processing may cause a risk of network over fitting, and require more training sets.

The embodiments of the present disclosure disclose image processing methods and systems thereof, which realize noise reduction and/or artifact removal of one or more material density images through iterative decomposing, noise reduction processing and/or artifact removal processing. A penalty function may be determined based on a convolution neural network, which can effectively avoid over fitting during neural network training and improve signal-to-noise ratios of the one or more material density images.

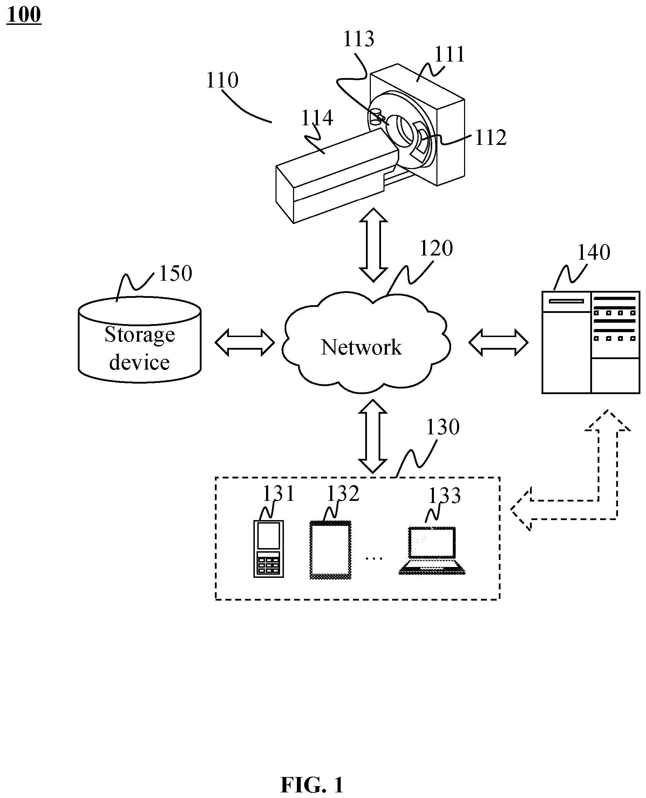

FIG. 1 is an exemplary schematic diagram illustrating an exemplary application scenario of an image processing system according to some embodiments of the present disclosure.

In some embodiments, an image processing system 100 is used to obtain one or more CT images (or X-ray images), and decompose the one or more CT images (or X-ray images) through matrix inversion material decomposition or the least square decomposition to obtain one or more initial material density images. In some embodiments, the image processing system 100 may be used to perform noise reduction processing and/or artifact removal processing on the one or more initial material density images to obtain one or more target material density images.

As shown in FIG. 1, in some embodiments, the image processing system 100 may include an imaging device 110, a network 120, a terminal 130, a processing equipment 140, and a storage device 150.

The imaging device 110 may be used to scan a target object or a portion thereof located within the imaging device 110's detection area and generate an image related to the target object or a portion thereof. In some embodiments, the target object may include a body, a substance, or the like, or any combination thereof. In some embodiments, the target object may include a specific part of the body, such as the head, chest, abdomen, etc., or any combination thereof. In some embodiments, the target object may include a specific organ, such as the heart, esophagus, trachea, bronchus, stomach, gallbladder, small intestine, colon, bladder, ureter, uterus, fallopian tube, etc. In some embodiments, the target object may include a patient or any other medical experiment object (for example, an animal such as a mouse).

In some embodiments, the imaging device 110 may include an X-ray scanner, a Computer Tomography (CT) instrument, etc. In some embodiments, the imaging device 110 may include a gantry 111, a detector 112, a scanning area 113, and a scanning table 114. The target object may be placed on the scanning table 114 for scanning. The gantry 111 may be used to support the detector 112. The detector 112 may be used to detect radiation beams. The scanning area 113 may refer to an imaging area for detecting the radiation beams.

In some embodiments, the detector 112 may include one or more detector units. In some embodiments, the detector units may include a single row of detectors and/or a plurality of rows of detectors. In some embodiments, the detector units may include a scintillation detector (for example, a cesium iodide detector), other detectors, or the like. In some embodiments, the gantry 111 may rotate, for example, in CT imaging, the gantry 111 may rotate clockwise or counterclockwise around a rotation axis. In some embodiments, the imaging device 110 may further include a radiation scanning source that may rotate with the gantry 111. The radiation scanning source can send the radiation beams (for example, X-rays) to the target object, which are attenuated by the target object and detected by the detector 112, thereby generating image signals. In some embodiments, the scanning table 114 may be movably arranged in front of the gantry 111 and parallel to the ground. The scanning table 114 may move in and out of the scanning area 113, and the scanning table 114 may also move in a vertical direction to adjust a distance between the target object on the scanning table and the detector 112 (or a scanning center) when entering the scanning area 113 to scan the target object within the scanning range.

The network 120 may include any suitable network capable of facilitating the exchange of information and/or data of the image processing system 100. In some embodiments, one or more components of the image processing system 100 (for example, the imaging device 110, the terminal 130, the processing device 140, the storage device 150, etc.) may exchange information and/or data with one or more components of the image processing system 100 through the network 120. For example, the processing device 140 may obtain scanning data from the imaging device 110 through the network 120.

In some embodiments, the network 120 may include a public network (such as the Internet), a private network (such as a local area network (LAN), a wide area network (WAN), a wired network (such as Ethernet), a wireless network (such as an 802.11 network, a wireless Wi-Fi network, etc.), a cellular network (such as a long-term evolution (LTE) network), a frame relay network, a virtual private network (VPN), a satellite network, a telephone network, a router, a hub, a server computer, etc. For example, the network 120 may include a wired network, an optical fiber network, a telecommunications network, a local area network, a wireless local area network (WLAN), a metropolitan area network (MAN), a public switched telephone network (PSTN), and Bluetooth™ Network, ZigBee™ Network, near field communication (NFC) network, etc. In some embodiments, network 120 may include one or more network access points. For example, the network 120 may include wired and/or wireless network access points, such as base stations and/or Internet switching points, through the access points, one or more components of the image processing system 100 may connect to the network 120 to exchange data and/or information.

In some embodiments, the terminal 130 may interact with other components in the image processing system 100 through the network 120. For example, the terminal 130 may send one or more control instructions to the imaging device 110 through the network 120 to control the imaging device 110 to scan the target object according to the instructions. As another example, the terminal 130 may receive the one or more material density images determined by the processing device 140 through the network 120 and display the one or more material density images for the operator to analyze and confirm.

In some embodiments, the terminal 130 may include a mobile device 131, a tablet 132, a laptop 133, or any combination thereof. In some embodiments, the mobile device 131 may include a smart home device, a wearable device, a mobile device, a virtual reality device, an augmented reality device, or any combination thereof. In some embodiments, the smart home device may include an intelligent lighting device, an intelligent electrical appliance control device, an intelligent monitoring device, an intelligent television, an intelligent ray machine, a walkie talkie, or any combination thereof. In some embodiments, mobile devices may include mobile phones, personal digital assistants (PDAs), game devices, navigation devices, POS devices, laptops, tablets, desktops, and the like, or any combination thereof. In some embodiments, the virtual reality device and/or augmented reality device may include a virtual reality helmet, virtual reality glasses, virtual reality patch, augmented reality helmet, augmented reality glasses, augmented reality patch, etc., or any combination thereof. For example, the virtual reality device and/or augmented reality device may include Google Glass™, Oculus Rift™, HoloLens™ or Gear VR™, etc.

In some embodiments, the terminal 130 may be part of the processing device 140. In some embodiments, the terminal 130 may be integrated with the processing device 140 as a console of the imaging device 110. For example, the user/operator of the image processing system 100 (for example, a doctor or nurse) may control the operation of the imaging device 110 through the operation platform, such as scanning the target object, controlling movement of the scanning table 114, training the image processing model, obtaining the one or more material density images using the image processing model, etc.

The processing device 140 may process data and/or information obtained from the imaging device 110, the terminal 130, and/or the storage device 150. For example, the processing device 140 may process image information detected by the imaging device 110 to obtain one or more CT images or one or more X-ray images. As another example, the processing device 140 may process the one or more CT images or the one or more X-ray images including information of a plurality of energy segments to obtain one or more material density images of one or more materials.

In some embodiments, the processing device 140 may be a single server or a group of servers. The group of servers may be centralized or distributed. In some embodiments, the processing device 140 may be local or remote. For example, the processing device 140 may access information and/or data from the imaging device 110, the terminal 130, and/or the storage device 150 through the network 120. As another example, the processing device 140 may be directly connected to the imaging device 110, the terminal 130, and/or the storage device 150 to access information and/or data. In some embodiments, the processing device 140 may be implemented on a cloud platform. For example, the cloud platform may include one or more combinations of private cloud, public cloud, hybrid cloud, community cloud, distributed cloud, cross cloud, multi cloud, etc.

The storage device 150 may store data (e.g., scanning data of a target object), instructions, and/or any other information. In some embodiments, the storage device 150 may store data obtained from the imaging device 110, the terminal 130, and/or the processing device 140. For example, the storage device 150 may store scanning data of a target object obtained from the imaging device 110. In some embodiments, the storage device 150 may store data and/or instructions that the processing device 140 may execute or use to perform the exemplary methods described in the present disclosure.

In some embodiments, the storage device 150 may include one or more combinations of mass memory, removable memory, volatile read-write memory, read-only memory (ROM), etc. The mass storage may include disk, optical disk, solid state disk, mobile storage, etc. Removable memory can include flash drives, floppy disks, optical disks, memory cards, zip disks, magnetic tapes, etc. Volatile read-write memory may include random access memory (RAM). RAM can include dynamic random access memory (DRAM), dual data rate synchronous dynamic random access memory (DDR-SDRAM), static random access memory (SRAM), silicon controlled random access memory (T-RAM), zero capacitance random access memory (Z-RAM), etc. ROM can include mask read only memory (MROM), programmable read only memory (PROM), erasable programmable read only memory (EPROM), electrically erasable programmable read only memory (EEPROM), optical disk read only memory (CD-ROM), digital multi-function optical disk, etc. In some embodiments, the storage device 150 may be implemented through the cloud platform described in this application.

In some embodiments, the storage device 150 may be connected to the network 120 to achieve communication with one or more components in the image processing system 100 (for example, the processing device 140, the terminal 130, etc.). One or more components in the image processing system 100 may read data or instructions in the storage device 150 through the network 120. In some embodiments, the storage device 150 may be part of the processing device 140 or may be independent and directly or indirectly connected to the processing device 140.

It should be noted that the above description of the image processing system 100 is for illustrative purposes only and is not intended to limit the scope of the present disclosure. For those skilled in the art, various modifications and modifications may be made according to the application. However, these changes and modifications do not depart from the scope of this application. For example, the image processing system 100 may also include a display device for outputting and displaying material density images processed by the processing device 140. For another example, the imaging device 110, the processing device 140, and the terminal 130 may share the same storage device 150, or they may have their own storage devices.

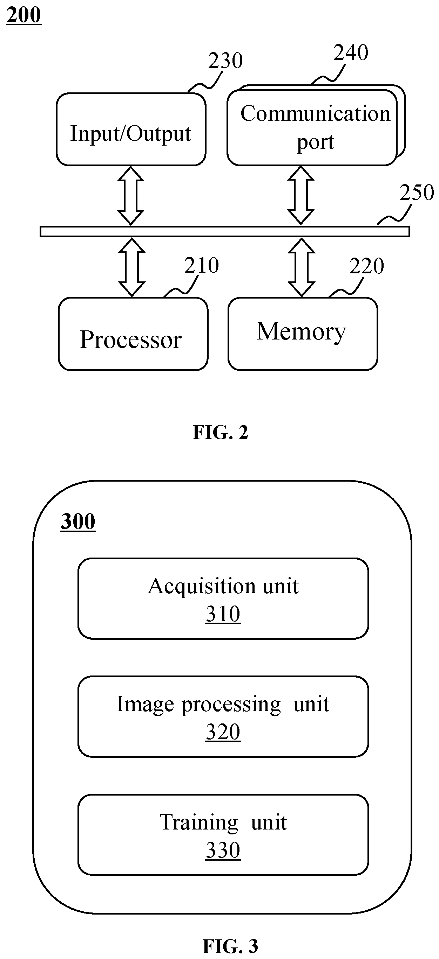

FIG. 2 is a schematic diagram of hardware and/or software of an exemplary computing device according to some embodiments of the present disclosure.

As shown in FIG. 2, in some embodiments, a computing device 200 may include a processor 210, a memory 220, an input/output interface 230, a communication port 240, and a bus 250.

The processor 210 may execute calculation instructions (e.g., program codes) and perform functions of the image processing system 100 described in the present disclosure. The calculation instructions may include programs, objects, components, data structures, processes, modules and functions (e.g., specific functions described in the present disclosure). For example, the processor 210 may process the one or more initial material density images obtained from any component of the image processing system 100. In some embodiments, the processor 210 may include a microcontroller, microprocessor, reduced instruction set computer (RISC), application specific integrated circuit (ASIC), application specific instruction set processor (ASIP), central processing unit (CPU), graphics processing unit (GPU), physical processing unit (PPU), microcontroller unit, digital signal processor (DSP), field programmable gate array (FPGA), advanced RISC machine (ARM), programmable logic devices, and any circuit and processor that can perform one or more functions, or any combination thereof. For illustration only, the computing device 200 in FIG. 2 describes only one processor, but it should be noted that the computing device 200 in the present disclosure may also include a plurality of processors.

The memory 220 may store data/information obtained from any other component of the image processing system 100. In some embodiments, memory 220 may include mass memory, removable memory, volatile read and write memory, read only memory (ROM), etc., or any combination thereof.

The input/output interface 230 may be used to input or output signals, data, or information. In some embodiments, the input/output interface 230 may enable the user to communicate with components in the image processing system 100 (for example, the imaging device 110). In some embodiments, the input/output interface 230 may include an input device and an output device. An exemplary input device may include any combination of one or more of a keyboard, a mouse, a touch screen, a microphone, and the like. An exemplary output device may include a display device, a speaker, a printer, a projector, and the like, or any combination thereof. An exemplary display device may include any combination of one or more of a liquid crystal display (LCD), a light emitting diode (LED) based display, a flat panel display, a curved surface display, a television device, a cathode ray tube (CRT), or the like.

The communication port 240 may be connected to the network for data communication. The connection may be a wired connection, a wireless connection, or a combination of the two. In some embodiments, the communication port 240 may be a standardized port, such as RS232, RS485, etc. In some embodiments, the communication port 240 may be a specially designed port. For example, the communication port 240 may be designed according to digital imaging and medical communication protocol (DICOM).

The bus 250 may include hardware and/or software for coupling components of the computing device 200 to each other. In some embodiments, the bus 250 may include: Data Bus, Address Bus, Control Bus, Expansion Bus, Local Bus. In some embodiments, the bus 250 may include an Accelerated Graphics Port (AGP) or other graphics bus, an Extended Industry Standard Architecture (EISA) bus, a Front Side Bus (FSB), Hyper Transport (HT) interconnect, Industry Standard Architecture (ISA) bus, wireless bandwidth (InfiniBand) interconnection, Low Pin Count (LPC) bus, memory bus, Micro Channel Architecture (MCA) bus, Peripheral Component Interconnect (PCI) bus, PCI Express (PCI-X) bus, Serial Advanced Technology Attachment (SATA) bus, Video Electronics Standards Association local bus (VLB), etc., or any combination thereof. In some embodiments, the bus 250 may include one or more buses. Although specific buses are described and shown in the embodiments of the present disclosure, this application considers any suitable bus or interconnection.

FIG. 3 is a schematic diagram of an exemplary image processing system according to some embodiments of the present disclosure.

As shown in FIG. 3, in some embodiments, the image processing system 300 may include an acquisition unit 310 and an image processing unit 320. In some embodiments, one or more modules in the image processing system 300 may be interconnected, the connection may be wireless or wired. At least a portion of the image processing system 300 may be implemented on the processing device 140 or the terminal 130 shown in FIG. 1.

The acquisition unit 310 may be used to obtain one or more initial material density images. In some embodiments, the acquisition unit 310 may be configured to obtain one or more images to be processed. In some embodiments, the acquisition unit 310 may determine the one or more initial material density images based on the one or more images to be processed.

The image processing unit 320 may be configured for image processing. In some embodiments, the image processing unit 320 inputs the one or more initial material density images into a trained image processing model to obtain the one or more target material density images. In some embodiments, the image processing model includes a neural network unit and an iterative decomposition unit.

In some embodiments, the image processing model may determine the one or more target material density images by performing one or more iterative operations, noise reduction processing and/or artifact removal processing on the one or more initial material density images at the same time. In some embodiments, the image processing unit 320 simultaneously performs decomposition processing, the noise reduction processing and/or the artifact removal processing on the one or more initial material density images using one or more iterative operations through the image processing model.

In some embodiments, the neural network unit is used to perform the noise reduction processing and/or the artifact removal processing on the one or more initial material density images. In some embodiments, the neural network unit includes an image conversion model constructed based on a convolutional neural network. In some embodiments, the iterative decomposition unit is configured to iteratively update the one or more initial material density images. In some embodiments, the iterative decomposition unit may include an objective function including a data fidelity term and a data penalty term determined based on the image conversion model. In some embodiments, the objective function may include:

$$arg \min_{x}[X(\boldsymbol{\mu},\boldsymbol{x}) + Y(\boldsymbol{x},\boldsymbol{u})], \qquad (1)$$

where, $X(\boldsymbol{\mu}, \boldsymbol{x})$ is the data fidelity item; $\mu$ represents the one or more images to be processed; $\boldsymbol{x}$ represents one or more material density images to be updated, and initial values of *x* is the one or more initial material density images; *Y*(*x, u*) is the data penalty term determined based on the image conversion model; *u* represents one or more material decomposition images obtained by using the image conversion model.

In some embodiments, for one or more iterations of the noise reduction processing and/or the artifact removal processing, an input of the neural network unit in an initial iteration of the one or more iterations includes the one or more initial material density images, and an input of the neural network unit in an *m*-th iteration of the one or more iterations may include one or more updated material density images determined by the iterative decomposition unit in an (*m*-1)th iteration of the one or more iterations, *m* being a positive integer greater than 1. In some embodiments, for one or more iterations of the decomposition processing, an input of the iterative decomposition unit in an *n*th iteration of the one or more iterations includes one or more material decomposition images output by the neural network unit in the *n*th iteration, *n* being a positive integer.

In some embodiments, the one or more target material density images may include material density images output by the trained image processing model when a first iteration termination condition is satisfied. The first iteration termination condition may include that a count of iteration times of the iterative operation reaches a first preset count, and/or a value of a first loss function in the iterative operation is less than or equal to a first preset loss function threshold.

In some embodiments, the image processing unit 320 may be configured to adjust the one or more target material density images based on one or more instructions. In some embodiments, the image processing unit 320 can determine weight values of the one or more target material density images and one or more reference material density images based on the one or more instructions, and determine, based on the weight values, a weighted sum the one or more target material density images and the one or more reference material density images to obtain one or more adjusted target material density images.

In some embodiments, the image processing model may include a plurality of subunits configured to perform the noise reduction processing and/or the artifact removal processing. Each of the sub units may be configured for different levels of the noise reduction processing and/or the artifact removal processing, respectively. In some embodiments, the image processing unit 320 may determine, based on the one or more instructions, one or more sub units that match the processing level(s), and determine processing results (of the one or more initial material density images) obtained by using the one or more sub units as the one or more adjusted target material density images.

In some embodiments, the image processing unit 320 may determine one or more execution parameters of the noise reduction processing and/or the artifact removal processing of the image processing model based on the one or more instructions, and determine the one or more adjusted target material density images using the image processing model based on the execution parameters. In some embodiments, the execution parameters may be configured to control the processing levels of the noise reduction processing and/or the artifact removal processing of the image processing model. In some embodiments, the execution parameters may be input by the user, or automatically determined by the system based on one or more of user data, image data, device parameters, historical processing parameters, etc.

In some embodiments, the image processing system 300 may include a training unit 330. The training unit 330 is configured to train an image processing model. In some embodiments, the training unit 330 is configured to update model parameters of an initial neural network unit, such as an initial image conversion model. For example, the training unit 330 obtains a first training set and uses the first training set to train the initial image conversion model to update model parameters of the initial image conversion model. The first training set includes sample pairs, each of the one or more sample pairs including a sample initial material density image and a corresponding label material density image. In some embodiments, the training unit 330 further adjusts the model parameters. Specifically, the training unit 330 inputs the one or more initial material density images into an updated image conversion model to obtain one or more sample material decomposition images; based on the one or more sample material decomposition images, the one or more initial material density images is updated by using the iterative decomposition unit. Further, a second training set is determined based on one or more updated sample initial material density images and one or more corresponding label material density images, and the updated image conversion model is further trained with the second training set to further adjust the model parameters. In some embodiments, the training unit 330 takes the one or more updated sample initial material density images as inputs of the updated image conversion model, and iteratively adjusting the model parameters of the initial image conversion model to obtain the trained image processing model.

In some embodiments, the training unit 330 is configured to iteratively adjust the model parameters of the initial image conversion model. When a second iteration termination condition is satisfied, the iterations may be terminated to obtain the trained image processing model. The second iteration termination condition may include that a count of iteration times reaches a second preset count, and/or a value of a second loss function in the iterations is less than or equal to a second preset loss function threshold.

In some embodiments, the acquisition unit 310, the image processing unit 320, and the training unit 330 may be set in a same or different processing devices. For example, the acquisition unit 310, the image processing unit 320, and the training unit 330 may be set in the processing device 140. As another example, the acquisition unit 310 and the image processing unit 320 may be set in the processing device 140, while the training unit 330 may be set in any other processing device other than the processing device 140.

It should be noted that the above description of the image processing system 300 is for illustrative purposes only and is not intended to limit the scope of the present application. For those skilled in the art, various modifications and modifications may be made according to the application. However, these changes and modifications do not depart from the scope of this application. For example, one or more units of the above image processing system 300 may be omitted or integrated into a single module. As another example, the image processing system 300 may include one or more additional modules, such as a storage module for data storage.

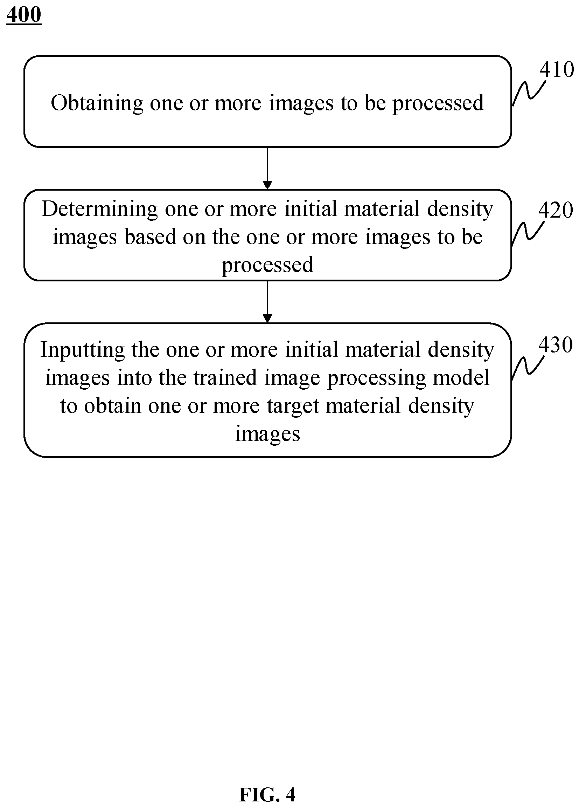

FIG. 4 is a flowchart of an exemplary image processing process according to some embodiments of the present disclosure.

In some embodiments, process 400 may be executed by the processing device 140. For example, the process 400 may be implemented as a set of instructions (e.g., an application) stored in a storage device (e.g., the storage device 150, the memory 220). In some embodiments, the processing device 140 may execute the set of instructions and may accordingly be directed to perform the process 400. The operations of the illustrated process presented below are intended to be illustrative. In some embodiments, the process 400 may be accomplished with one or more additional operations not described and/or without one or more of the operations discussed. Additionally, the order of the operations of process 400 illustrated in FIG. 4 and described below is not intended to be limiting.

In 410, one or more images to be processed may be obtained. In some embodiments, operation 410 may be performed by the processing device 140 or the acquisition unit 310.

In some embodiments, the one or more images to be processed may be one or more CT images or X-ray images obtained by scanning a target object through an imaging device (such as the imaging device 110). In some embodiments, the target object may include a patient or other medical experiment object (e.g., any other animal such as a mouse). In some embodiments, the target object may be a part of a patient or any other medical experiment object, including an organ and/or tissue, such as the heart, lungs, ribs, abdominal cavity, etc. In some embodiments, the one or more images to be processed may include at least a portion of the target object (such as human, animal), such as head, spine, neck, chest, lung, heart, stomach, blood vessels, soft tissue, tumor, nodule, etc. In some embodiments, the initial image may be a two-dimensional image and/or a three-dimensional image.

In some embodiments, the processing device 140 may control the imaging device to scan the target object at the same or different angles with rays of different energies to obtain the one or more images to be processed. The images to be processed may include two or more energy segments. In some embodiments, the one or more images to be processed may be one or more CT images generated by a photon counting CT device. In some embodiments, the one or more images to be processed may be one or more X-ray images.

In some embodiments, the processing device 140 may obtain the one or more images to be processed of the target object by reading from an imaging device, a database, or a storage device, or calling a data interface.

In 420, one or more initial material density images may be determined based on the one or more images to be processed. In some embodiments, the operation 420 may be performed by the processing device 140 or the acquisition unit 310.

The one or more initial material density images may refer to one or more material density images that has not been processed.

CT imaging may be attenuation coefficient imaging. X-rays have penetrability and can penetrate the target object (e.g., a life body, an object). Different tissues of the target object may have different absorption and transmittance of the X-rays. When the X-rays pass through the target object, different parts of the target object may have different attenuation of the X-rays. Therefore, by measuring attenuated X-rays, data of different parts of the target object may be obtained. After processing obtained data, a cross-section or stereo image of an inspected part of the target object may be reconstructed. Linear attenuation coefficients of the target object may be expressed as a linear combination of the one or more material density images and material decomposition of one or more materials. Since CT values of the one or more CT images have a linear relationship with the linear attenuation coefficients, the one or more CT images of the target object may be expressed as a linear combination of the one or more material density images and a material decomposition matrix of the material, as shown in Equation (2):

$$
\begin{pmatrix} \mu_{B1} \\ \mu_{B2} \\ \vdots \\ \mu_{BN} \end{pmatrix} = \begin{pmatrix} \mu_{B1,1} & \mu_{B1,2} & \cdots & \mu_{B1,M} \\ \mu_{B2,1} & \mu_{B2,2} & \cdots & \mu_{B2,M} \\ \vdots & \vdots & \vdots & \vdots \\ \mu_{BN,1} & \mu_{BN,1} & \cdots & \mu_{BN,M} \end{pmatrix} \begin{pmatrix} x_1 \\ x_2 \\ \vdots \\ x_M \end{pmatrix} \tag{2}
$$

where, $\mu_{BN}$ represents the one or more CT images in various energy segments (i.e., the one or more images to be processed), and B1, B2 and BN refer to each energy segment respectively; a matrix including $\mu_{B1,1}$, ..., $\mu_{BN,M}$, is the material decomposition matrix A, $\mu_{E,I}$ is a mass attenuation coefficient (i.e., a material density coefficient) of material I (I=1, 2, ..., M) in the material decomposition matrix A in an energy segment E (E=B1, B2, ..., BN), and M refers to a type of material; $x_M$ represents the one or more material density images corresponding to each material.

The one or more material density images may reflect density distribution of materials. In some embodiments, the material may include calcium, iodine, water, air, fat, soft tissue, blood vessels, etc., or any combination thereof. In some embodiments, the processing device 140 may determine the one or more initial material density images based on the one or more images to be processed. For example, the processing device 140 may bring the one or more images to be processed into Equation (2), and determine the one or more initial material density images by the matrix inversion material decomposition (e.g., double base decomposition, triple base decomposition, multi base material decomposition, etc.) or the least square decomposition. As another example, the processing device 140 may input the one or more images to be processed to the trained image processing model to obtain the one or more initial material density images. In some embodiments, the processing device 140 may obtain the one or more initial material density images using the iterative decomposition unit in the image processing model. For example, the processing device 140 may set the data penalty term of the objective function (1) to 0, substitute the one or more images to be processed into the data fidelity term of the objective function (1), and determine the one or more initial material density images by the matrix inversion material decomposition or the least square decomposition. In some embodiments, the image processing model may include an image decomposition unit for determining the one or more initial material density images.

In some embodiments, signal-to-noise ratios of the one or more initial material density images obtained by the matrix inversion decomposition or the least square decomposition of the one or more CT images (or the one or more X-ray images) including different energy segments may be degraded. In some embodiments, the signal-to-noise ratios of the one or more initial material density image may be improved by performing the noise reduction processing and/or the artifact removal processing on the one or more initial material density images. The signal-to-noise ratios may refer to ratios of signal to noise in images. The signal-to-noise ratio can reflect a level of noise (such as Gaussian noise, Poisson noise, etc.) in an image. The larger the signal-to-noise ratio of the image, the smaller the noise in the image.

In 430, the one or more initial material density images may be input into the trained image processing model to obtain the one or more target material density images. In some embodiments, operation 430 may be performed by the processing device 140 or the image processing unit 320.

The one or more target material density images may refer to the one or more material density images obtained after the noise reduction processing and/or the artifact removal processing. In some embodiments, the one or more target material density images may be used for analysis and diagnosis of diseases. In some embodiments, the processing device 140 may use the trained image processing model to decompose and perform the noise reduction processing and/or the artifact removal processing on the one or more initial material density images to obtain the one or more target material density images.

In some embodiments, the image processing model may include a neural network unit and an iterative decomposition unit.

In some embodiments, the neural network unit may be configured to perform the noise reduction processing and/or the artifact removal processing on the one or more material density images. In some embodiments, the neural network unit may include an image conversion model constructed based on a convolution neural network, a graph neural network, a depth neural network, or the like, or a combination thereof. In some embodiments, the neural network unit may include an image conversion model constructed based on the convolution neural network. In some embodiments, the input of the neural network unit may include one or more material density images with low signal-to-noise ratios, and the output of the neural network unit may include one or more material density images with higher signal-to-noise ratios and/or artifact removal. For example, the input of the neural network unit may be the one or more initial material density images, or one or more iteratively updated material density images through the iterative decomposition unit, and the output of the neural network unit may be the one or more material decomposition images (also known as one or more intermediate material density images) or the one or more target material density images.

In some embodiments, the iterative decomposition unit may be used to iteratively decompose and update the one or more material density images to balance signals and noises of the one or more material density images. For example, the iterative decomposition unit may be configured to balance signals and noises of the one or more material decomposition images to reduce the noises of the one or more material decomposition images, while ensuring that the useful signals of the one or more material decomposition images are not reduced or affected, so as to obtain the one or more updated material density images with lower noises. In some embodiments, the input of the iterative decomposition unit may include the one or more material decomposition images with relatively low signal-to-noise ratios output by the neural network unit, and the output of the iterative decomposition unit may include the one or more updated material density images with relatively high signal-to-noise ratios.

In some embodiments, the iterative decomposition unit may include a data fidelity term and a data penalty term. In some embodiments, the iterative decomposition unit may include an objective function (e.g., the objective function (1) that obtains a variate value when a sum of the data fidelity term and the data penalty term determined based on the image conversion model reaches a minimum value) including the data fidelity term and the data penalty term determined based on the image conversion model. In some embodiments, the iterative decomposition unit may balance noises between the one or more material decomposition images output by the neural network unit and the one or more material density images of the data fidelity term through the data penalty term to obtain the one or more material density images with higher signal-to-noise ratios.

In some embodiments, the image processing model may simultaneously perform decomposition processing, the noise reduction processing and/or the artifact removal processing on the one or more initial material density images using an iterative operation to determine the one or more target material density images. For example, the image processing model may input the one or more initial material density images into the neural network unit, and take the one or more processed material decomposition images output by the neural network unit as the input of the iterative decomposition unit, and then input the one or more updated material density images output by the iterative decomposition unit into the neural network unit again for processing. After several iterations using the iterative decomposition unit and the neural network unit, the one or more target material density images may be determined.

In some embodiments, for one or more iterations of the noise reduction processing and/or the artifact removal processing, an input of the neural network unit in an initial iteration of the one or more iterations may include the one or more initial material density images, and an input of the neural network unit in an m-th iteration of the one or more iterations may include one or more updated material density images determined by the iterative decomposition unit in an (m-1)th iteration of the one or more iterations, m being a positive integer greater than 1. Accordingly, for one or more iterations of the decomposition processing, an input of the iterative decomposition unit in an nth iteration of the one or more iterations may include one or more material decomposition images output by the neural network unit in the nth iteration, n being a positive integer. For example, in the one or more iterations through the image processing model, an input of the neural network unit in a 5th iteration may be one or more updated material density images obtained by the iterative decomposition unit in a 4th iteration; an input of the iterative decomposition unit in the 4th iteration may be the one or more material decomposition images output by the neural network unit in the 4th iteration.

In some embodiments, the image processing model may include a plurality of sub units (e.g., a plurality of neural network sub units) configure to perform the noise reduction processing and/or the artifact removal processing. In some embodiments, different sub units (e.g., neural network sub units) may be used for different levels of the noise reduction processing and/or the artifact removal processing. For example, the neural network units may include sub unit 1 and sub unit 2. After the sub unit 1 performs the noise reduction processing and/or the artifact removal processing on one or more material density images, the processing effect (e.g., denoising effect and/or artifact removal effect) of the obtained one or more material decomposition images may be more than 95% compared with the one or more material density images. After the sub unit 2 performs the noise reduction processing and/or the artifact removal processing on the one or more material density images, the processing effect (e.g., denoising effect and/or artifact removal effect) of the obtained one or more material decomposition images may be between 80% and 95% compared with the one or more material density images.

In some embodiments, the one or more target material density images may be one or more material density images determined by the image processing model when the first iteration termination condition is satisfied. For example, the one or more target material density images may be the one or more material decomposition images output by the neural network unit or the one or more material density images output by the iterative decomposition unit when the first iteration termination condition is satisfied.

In some embodiments, the first iteration termination condition may include at least one of the following: a count of iteration times of the iterative operation reaches a first preset count (e.g., the count of iteration times of the iterative operation reaches the first preset count), a value of a first loss function in the iterative operation is less than or equal to a first preset loss function threshold (e.g., less than or equal to the first preset loss function threshold), and the signal-to-noise ratio(s) of the one or more target material density images may be greater than a signal-to-noise ratio threshold. In some embodiments, the first loss function may include one or more differences between two groups of material decomposition images determined by the image processing model in two adjacent iterations respectively (i.e., the one or more material decomposition images output by the neural network unit in the two adjacent iterations) or one or more differences between two groups of updated material density images determined by the trained image processing model in two adjacent iterations (i.e., the one or more updated material density images output by the iterative decomposition unit in the two adjacent iterations).

In some embodiments, the first preset count may be any reasonable threshold representing the count of iteration times of the image processing model. Merely by way of example, the first preset count may be 2, 10, 100, 500, etc.

In some embodiments, the first preset loss function threshold may be any reasonable threshold for the difference (e.g., distance, or similarity, etc.) between two groups of material decomposition images or updated material density images determined by two adjacent iterations. In some embodiments, the first preset loss function threshold may be expressed as any reasonable numerical value type, such as percentage (such as 1%, 5%, etc.), or decimal (such as 0.01, 0.03, etc.), or fraction, or integer. In some embodiments, the difference between the two groups of the updated material density images determined by two adjacent iterations may include one or more differences or ratios of image quality values of the two groups of the updated material density images.

In some embodiments, the signal-to-noise ratio threshold may be a threshold for the signal-to-noise ratios of the one or more target material density images. Merely by way of example, the signal-to-noise ratio threshold may be 10dB, 20dB, 50dB, etc.

In some embodiments, the first iteration termination condition may be automatically determined by the system (e.g., the processing device 140) and/or input by the user. For example, the processing device may automatically determine a threshold of first iteration termination times based on data related to the iteration termination condition input by the user (e.g., a doctor), feedback data of the determined one or more target material density images, the user diagnostic habit and other user data, and/or the historical material decomposition processing data, historical diagnostic data, or the like.

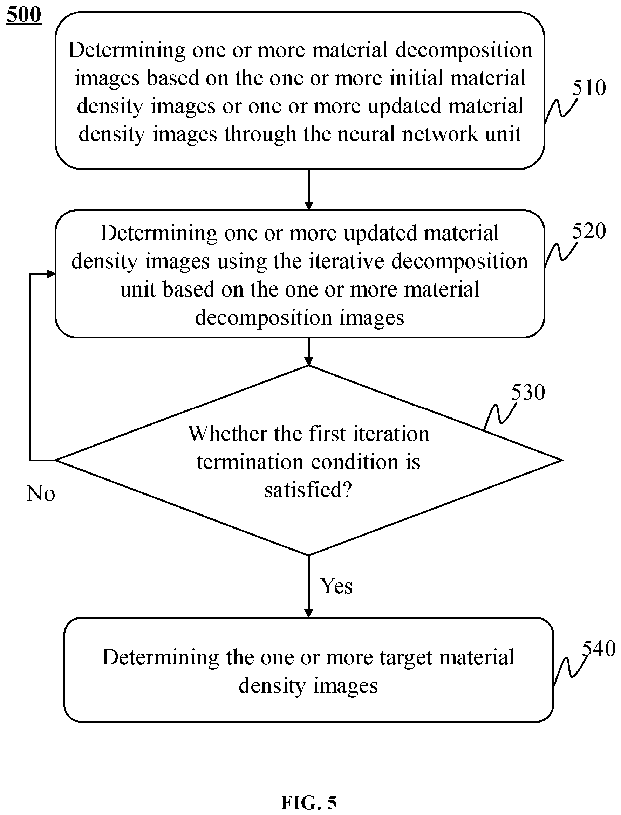

More descriptions of the iterations may be found elsewhere in the present disclosure (e.g., FIG. 5 and descriptions thereof).

In some embodiments, an instruction may be obtained through an operation interface, and the one or more target material density images may be to adjusted based on the instruction. The instruction may indicate a processing level of the noise reduction processing and/or the artifact removal processing corresponding to the one or more target material density images, i.e., a degree of the noise reduction processing and/or the artifact removal processing. For example, an instruction input by the user indicating the processing level of the noise reduction processing and/or the artifact removal processing corresponding to the one or more target material density images may be obtained through the operation interface of the terminal 130.

In some embodiments, one or more reference material density images may be obtained, weight values of the one or more target material density images and the one or more reference material density images may be determined based on the instruction, and a weighted sum of the one or more target material density images and the one or more reference material density images may be determined based on the weight values to obtain one or more adjusted target material density images.

In some embodiments, the one or more reference material density images may include one or more post processed target material density images. In some embodiments, the one or more reference material density images may be one or more material density images input by a user. In some embodiments, the one or more reference material density images may be one or more material density images obtained by an image processing model. In some embodiments, the weight values of the one or more target material density images and the one or more reference material density images may be determined according to the processing level of the noise reduction processing and/or the artifact removal processing that the one or more target material density images need to satisfy as indicated in the instruction. In some embodiments, respective weight values of the one or more target material density images and the one or more reference material density images may be determined based on a current processing level of the noise reduction processing and/or the artifact removal processing of the one or more target material density images. For example, ratios of weight values of the one or more target material density images to that of the one or more reference material density images may be 5:5, 3:7, 2:8, 6:4, etc.

In some embodiments, a processing sub unit that matches the processing level of the noise reduction processing and/or the artifact removal processing corresponding to the one or more target material density images may be determined based on the instruction, and one or more processing results of material density images obtained using the processing sub unit may be determined as the one or more adjusted target material density images.

Merely by way of example, if the image processing model includes three neural network sub units, which are respectively used for high (e.g., the processing degree of the noise reduction processing and/or the artifact removal processing of images reaches more than 80%), medium (e.g., the processing degree of the noise reduction processing and/or the artifact removal processing of images is between 50% and 80%), low (e.g., the degree of the noise reduction processing and/or the artifact removal processing of images is less than 50%) levels of the noise reduction processing and/or the artifact removal processing. If the user inputs a current processing level as "high" through the terminal 130, the system may input the one or more initial material density images into the neural network sub unit with the processing level "high", and determine the one or more material density images that satisfy the first iteration termination condition as the one or more adjusted target material density images. Alternatively, the system may take one or more current target material density images (e.g., the one or more target material density images obtained after inputting the one or more initial material density images into the image processing model) as new inputs, and input them into the neural network sub unit with the processing level "high", to iteratively perform decomposition and the noise reduction processing and/or the artifact removal processing again. One or more material density images that satisfy the first iteration termination condition may be determined as the one or more adjusted target material density images.

In some embodiments, the image processing unit 320 may determine the execution parameters of the noise reduction processing and/or the artifact removal processing of the image processing model based on an instruction, and determine the one or more adjusted target material density images using the image processing model based on the execution parameters. In some embodiments, the execution parameters may be configured to control the processing level of the image processing model for the noise reduction processing and/or the artifact removal processing. For example, the image processing model may include a parameter setting unit for determining the processing degree of the noise reduction processing and/or the artifact removal processing of the neural network unit. After values of the execution parameters are determined based on a user instruction, the values may be input with the one or more initial material density images or the one or more current target material density images (e.g., the one or more target material density images obtained after inputting the one or more initial material density images into the image processing model) into the image processing model to obtain the one or more adjusted target material density images.

In some embodiments, the execution parameters may be provided by the user. For example, the user may manually set the execution parameters of the image processing model when the model is trained or used. In some embodiments, the execution parameters may be automatically determined by the system based on one or more of user data (e.g., diagnostic habits of doctors, data related to the execution parameters input by the user historically, etc.), image data (e.g., the one or more initial material density images, one or more intermediate material density images, and/or noise and/or artifact degree of the one or more target material density images, etc.), device parameters (e.g., performance parameters, service life, service time, etc., of the imaging device that obtains the one or more images to be processed or the one or more initial material density images), historical processing parameters (e.g., the execution parameters used in the historical acquisition of the one or more target material density images, an iteration termination condition, a processing level, etc.), or the like, or a combination thereof.

FIG. 5 is a flowchart of an iterative calculation process of an exemplary image processing model according to some embodiments of the present disclosure.

In some embodiments, process 500 may be executed by the processing device 140. For example, the process 500 may be implemented as a set of instructions (e.g., an application) stored in a storage device (e.g., the storage device 150, the memory 220). In some embodiments, the processing device 140 may execute the set of instructions and may accordingly be directed to perform the process 500. The operations of the illustrated process presented below are intended to be illustrative. In some embodiments, the process 500 may be accomplished with one or more additional operations not described and/or without one or more of the operations discussed. Additionally, the order of the operations of process 500 illustrated in FIG. 5 and described below is not intended to be limiting.

In 510, one or more material decomposition images may be determined based on the one or more initial material density images or the one or more updated material density images through the neural network unit.

The one or more material decomposition images may be obtained by the noise reduction processing and/or the artifact removal processing through the neural network unit in one iteration. In some embodiments, the processing device 140 may input the one or more initial material density images or the one or more updated material density images (e.g., the one or more material density images updated by the iterative decomposition unit) to the neural network unit to obtain the one or more material decomposition images output by the neural network unit.

In some embodiments, the neural network unit may include a convolution layer and a full connection layer. The convolution layer may be configured to obtain local features of input data, and the full connection layer may be configured to obtain global features of the input data. For example, the convolution layer of the neural network unit may obtain local features $\{x_{1,1}, x_{1,2}, ... , x_{1,M}\}$ of one or more initial material density images $x_1$ based on the one or more initial material density images $x_1$, where $x_{1,1}$ represents a correlation between material 1 and the material 1 itself, $x_{1,2}$ represents a correlation between the material 1 and material 2, and $x_{1,M}$ represents a correlation between the material 1 and material $M$. In some embodiments, the full connection layer may reassemble the local features by means of weight matrix or summation operation to obtain corresponding one or more material decomposition images $u_1$. The full connection layer may integrate the local features of different materials. In some embodiments, the convolution layer may include one or more convolution kernels.

In some embodiments, the one or more initial material density images and/or the one or more updated material density images are represented by $x$, and may include one or more images. For example, $x$ may include $M$ material density images $x_1, x_2, ..., x_M$ representing $M$ different materials, the $M$ material density images $x_1, x_2, ..., x_M$ may be decomposed based on the one or more CT images or the one or more X-ray images. In some embodiments, the $M$ material density images may be input into the neural network unit respectively or at the same time to obtain corresponding $M$ material decomposition images $u_1, u_2, ..., u_M$. For example, the one or more material decomposition images $u_1$ may be obtained by integrating the local features $x_{1,1}, x_{2,1}, ... , x_{M,1}$ representing the correlation between each material in $M$ materials and material 1, the one or more material decomposition images $u_2$ may be obtained by integrating the local features $x_{1,2}, x_{2,2}, ... , x_{M,2}$ representing the correlation between each material in $M$ materials and material 2, and the one or more material decomposition images $u_M$ may be obtained by integrating the local features $x_{1,M}, x_{2,M}, ... , x_{M,M}$ representing the correlation between each material in $M$ materials and material $M$.

In some embodiments, the neural network unit may include a convolution layer, and the image processing model may input $M$ material density images into the neural network unit at the same time. After obtaining an autocorrelation feature image and a cross-correlation feature image of each material density image through the convolution layer, the image processing model may combine, for each material, through the full connection layer, the autocorrelation feature image representing a correlation between the material and the material itself and the cross-correlation feature image representing correlations between other materials and the material (e.g., for the material 1, a corresponding feature image may be: $x_{1,1}, x_{2,1}, ... , x_{M,1}$) to output $M$ material decomposition images after the noise reduction processing and/or the artifact removal processing. In some embodiments, the autocorrelation feature image may represent correlation features between each material density image and the each material density image itself, and the cross-correlation feature image may represent correlation features between each material density image and other material density images in the $M$ material density images. In some embodiments, the neural network unit may include a plurality of convolution layers (e.g., $M$ convolution layers), and the image processing model may input the $M$ material density images into the neural network unit respectively. Each convolution layer may calculate the autocorrelation feature image and the cross-correlation feature image of a corresponding material density image, and then output $M$ material decomposition images after the noise reduction processing and/or the artifact removal processing through the full connection layer.

In some embodiments, the neural network unit may include a plurality of sub units, different sub units may be used for different levels of the noise reduction processing and/or the artifact removal processing. In some embodiments, the neural network unit may determine the processing level of the noise reduction processing and/or the artifact removal processing for the one or more material density images based on user input instruction(s).

In 520, one or more updated material density images may be determined by using the iterative decomposition unit based on the one or more material decomposition images.

In some embodiments, the processing device 140 may input the one or more material decomposition images to the iterative decomposition unit to obtain the one or more updated material density images output by the iterative decomposition unit. The one or more updated material density images may balance useful signals and noises of the one or more material decomposition images, so that the noises of the one or more material density images may be reduced while keeping the useful signals in the image unaffected or unreduced, and the processing effect of the noise reduction processing and/or the artifact removal processing of the one or more material density images is better. In some embodiments, the iterative decomposition unit may include an objective function including the data fidelity term and the data penalty term determined based on the neural network unit (such as the image conversion model). In some embodiments, the objective function of the iterative decomposition unit (e.g., Equation (1) described in connection with FIG. 3) may obtain variate values when the sum of the data fidelity term and the data penalty term determined based on the image conversion model reaches the minimum value.

In some embodiments, the data fidelity term in the objective function may be configured to force the linear combination (e.g., $Ax$, where A represents the material decomposition matrix) of volume fractions $x$ to approximate one or more CT images (or X-ray images) $\mu$. The data penalty term may be configured to balance the one or more material decomposition images output by the neural network unit and the one or more material density images of the data fidelity term, making noises of the one or more updated material density images lower than noises of the one or more material decomposition images output by the neural network unit, and ensuring that image quality (such as useful signals) of the one or more updated material density images is not affected or reduced. In some embodiments, the initial value of $x$ may be an initialized value before the iterative calculation starts, the material decomposition matrix A may be related to energy segments of the one or more material decomposition images and types of materials to be decomposed. In some embodiments, penalty factors in the data penalty term may be related to the processing level of the noise reduction processing and/or the artifact removal processing required for the one or more material density images, the materials, or the like.

It may be understood that the above objective function (1) is only an example. In some embodiments, the iterative decomposition unit may use other reasonable objective functions to realize the update iteration of the one or more material density images, which is not limited in the present disclosure.

In some embodiments, the processing device 140 may input the one or more updated material density images into the neural network unit again to determine the one or more material decomposition images. For example, the one or more updated material density images determined by the iterative decomposition unit in a ($n$-1)th iteration may be used as input data and input to the neural network unit again to obtain the one or more material decomposition images output by the neural network unit in an $n$th iteration $u^{(n)}$. By inputting the one or more updated material density images into the neural network unit again for calculation, noises and/or artifacts may be further reduced or removed, and useful information in the one or more images may be retained as much as possible.

In 530, whether the first iteration termination condition is satisfied may be determined.

In some embodiments, the processing device 140 may determine whether a result of this iterative operation satisfies the first iteration termination condition. If the first iteration termination condition is satisfied, the process may proceed to 540 to determine the one or more updated material density images output from this iterative operation as the one or more target material density images. If the first iteration termination condition is not satisfied, the one or more updated material density images may be used as input data of the neural network unit, the process may proceed back to 510, and operations 510 to 520 may be performed again until the first iteration termination condition is met. More descriptions of the first iteration termination condition may be found elsewhere in the present disclosure (e.g., operation 430 of FIG. 4 and descriptions thereof).

In 540, one or more target material density images may be determined.

In some embodiments, the processing device 140 may determine the one or more updated material density images output when the first iteration termination condition is satisfied as the one or more target material density images. In some embodiments, the processing device 140 may determine whether the one or more target material density images satisfy one or more preset conditions (e.g., whether signal-to-noise ratios of the one or more target material density images are greater than a preset signal-to-noise ratio threshold or whether image qualities of the one or more target material density images are higher than an image quality threshold, or whether the one or more target material density images satisfy the user instruction). If the preset conditions are not satisfied, the one or more target material density images may be post processed or processed through the image processing model again. In some embodiments, whether the preset conditions are satisfied may be determined based on one or more instructions obtained through the operation interface. If the instruction(s) (indicating the processing level of the noise reduction processing and/or the artifact removal processing) corresponding to the one or more target material density images are satisfied, one or more current target material density images (e.g., the one or more target material density images obtained after the one or more initial material density images are input into the image processing model) may be determined as one or more final target material density images. Otherwise, the one or more current target material density images may be adjusted based on the instruction(s) to obtain one or more adjusted target material density images.

Merely by way of example, before the iterative operation, the one or more initial material density images $x^{(0)}$ may be input to the neural network unit to obtain output of the neural network unit, i.e., the one or more material decomposition images $u^{(1)}$. The one or more material decomposition images $u^{(1)}$ may be input to the iterative decomposition unit, the iterative decomposition unit may perform a first iteration for the objective function. After the first iteration, the one or more updated material density images $x^{(1)}$ may be obtained. Then the one or more updated material density images $x^{(1)}$ may be input to the neural network unit to obtain the output of the neural network unit, i.e., the one or more material decomposition images $u^{(2)}$. The one or more material decomposition images $u^{(2)}$ may be input to the iterative decomposition unit, the iterative decomposition unit may perform a second iteration for the objective function. After the second iteration, the one or more updated material density images $x^{(2)}$ may be obtained. Then the one or more updated material density images $x^{(2)}$ may be input to the neural network unit to obtain the output of the neural network unit, i.e., the one or more material decomposition images $u^{(3)}$. Similarly, in an nth iteration, using the one or more material decomposition images $u^{(n)}$ determined by the neural network unit, the one or more updated material density images $x^{(n)}$ may be output through an iterative model decomposition unit (i.e., the iterative decomposition unit). After at least one iteration, until the first iteration termination condition is satisfied, the iterative operation may be terminated, and one or more updated material density images $x^{(n)}$ may be determined as the one or more target material density images.

In some embodiments, the one or more target material density images may be used to determine distributions of material densities at a target site, so as to facilitate the diagnosis of diseases at the target site and the discovery of unknown diseases.

It should be noted that the above description of the process 400 and/or the process 500 is provided for illustrative purposes only and is not intended to limit the scope of the present disclosure. For those skilled in the art, various changes and modifications may be made according to the description of the present disclosure. However, these changes and modifications do not depart from the scope of the present disclosure. In some embodiments, the process 400 and/or the process 500 may include one or more additional operations, or one or more of the above operations may be omitted. For example, the process 500 may include one or more additional operations to determine the one or more material density images.

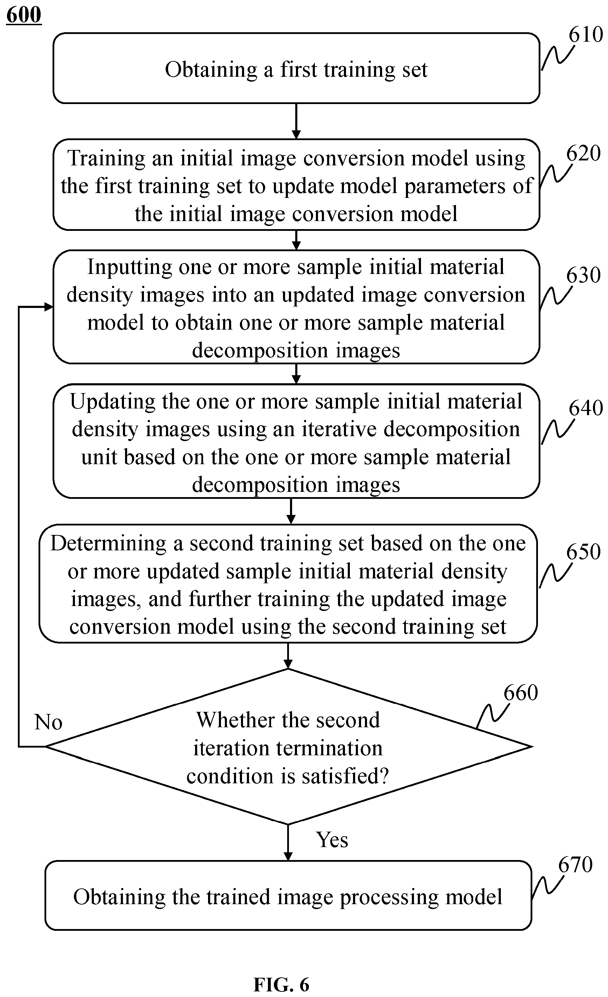

FIG. 6 is a flowchart of an exemplary image processing model determination process according to some embodiments of the present disclosure.

In some embodiments, process 600 may be executed by the processing device 140. For example, the process 600 may be implemented as a set of instructions (e.g., an application) stored in a storage device (e.g., the storage device 150, the memory 220). In some embodiments, the processing device 140 may execute the set of instructions and may accordingly be directed to perform the process 600. The operations of the illustrated process presented below are intended to be illustrative. In some embodiments, the process 600 may be accomplished with one or more additional operations not described and/or without one or more of the operations discussed. Additionally, the order of the operations of process 600 illustrated in FIG. 6 and described below is not intended to be limiting. In some embodiments, the process 400, the process 500, and the process 600 may be performed by different processing devices. For example, the process 400 and the process 500 may be executed by the processing device 140, while the process 600 may be executed by any other processing device other than the processing device 140.

In 610, a first training set may be obtained.

The first training set may include initial training data for training a neural network unit. In some embodiments, the first training set may include one or more sample pairs, and each of the one or more sample pairs may include a sample initial material density image and a corresponding label material density image. In some embodiments, the sample initial material density image may refer to an unprocessed material density image obtained based on a sample image (such as a CT images or X-ray image) (of the same or different scanning object as the target object) to be processed. In some embodiments, the label material density image may refer to a density image obtained after performing the noise reduction processing and/or the artifact removal processing on an initial material density image. A signal-to-noise ratio of the label material density image may be greater than a signal-to-noise ratio of the initial material density image. In some embodiments, one or more sample initial material density images may be obtained by performing the matrix inversion decomposition or the least square decomposition on the one or more images to be processed, and one or more label material density images may be obtained by performing the noise reduction processing and/or the artifact removal processing on the one or more sample initial material density images. In some embodiments, signal-to-noise ratios of the one or more label material density image may be higher than a signal-to-noise ratio threshold of the one or more material density images. For example, the signal-to-noise ratio threshold may be 1dB, 2dB, 5dB, etc.

In some embodiments, the one or more sample initial material density images may be used as the input data of the neural network unit during training, and the one or more label material density images may be used as desired output data of the neural network unit.

In some embodiments, the processing device (or the training unit 330) may obtain a first training set previously stored in the storage device. In some embodiments, the processing device (or the training unit 330) may obtain the first training set through one or more interfaces (e.g., one or more program interfaces, one or more data interfaces, one or more transmission interfaces, or the like).

In 620, the initial image conversion model may be trained using the first training set to update the model parameters of the initial image conversion model.

The initial image conversion model may refer to an untrained image conversion model (e.g., the initial neural network unit). In some embodiments, the processing device may build the initial image conversion model based on the convolutional neural network to predict one or more material decomposition images according to one or more material density images, and use the first training set to train the initial image conversion model to determine the model parameters of the initial image conversion model, so as to update the model parameters of the initial image conversion model. In some embodiments, the processing device may use any reasonable and feasible training method (e.g., a gradient descent method) to train the initial image conversion model to update the model parameters of the initial image conversion model and obtain the updated image conversion model, which is not limited in the present disclosure.

In 630, the one or more sample initial material density images may be input into the updated image conversion model to obtain one or more sample material decomposition images.

A sample material decomposition image may be an image with a relatively high signal-to-noise ratio predicted by the updated image conversion model. In some embodiments, the processing device may input a plurality of sample initial material density images into the updated image conversion model, and obtain a plurality of sample material decomposition images corresponding to the sample initial material density images.

In 640, the one or more sample initial material density images may be updated using the iterative decomposition unit based on the one or more sample material decomposition images.

In some embodiments, the processing device may input the one or more sample material decomposition images to the iterative decomposition unit, and obtain one or more updated initial material density images through an iteration operation. For example, the iterative decomposition unit may calculate and obtain the one or more updated initial material density images based on the one or more sample material decomposition images based on the objective function (1) including the data fidelity term and the data penalty term determined based on the image conversion model. Updating of the one or more initial material density images may be similar to updating of the one or more material density images in the 520, and more descriptions may be found elsewhere in the present disclosure (e.g., operation 520 of FIG. 5 and descriptions thereof). In some embodiments, the process of updating the one or more initial material density images/material density images using the iterative decomposition unit in operation 640 and operation 520 may include only one iteration of the objective function.

In 650, a second training set may be determined based on the one or more updated sample initial material density images, and the updated image conversion model may be further trained using the second training set.

In some embodiments, the processing device may take one or more updated sample initial material density images and the one or more corresponding label material density images as the second training set, and further train the updated image conversion model using the second training set. In some embodiments, the one or more updated sample initial material density images may be used as input data of the updated image conversion model, and the one or more corresponding label material density images may be used as expected output data of the updated image conversion model.

In some embodiments, the processing device may use a same or different training method as that used in training the initial image conversion model based on the first training set in 630, and train the updated image conversion model based on the second training set to obtain an updated neural network unit.

In 660, whether a second iteration termination condition is satisfied may be determined.

In some embodiments, operations 610-620 may be regarded as a process of updating the model parameters of the initial image conversion model, and operations 630-650 may be regarded as a process of further adjusting the model parameters. The processing device may take the one or more updated sample initial material density images as input of the updated image conversion model, and iteratively perform the process of further adjusting the model parameters to obtain the trained image processing model. In some embodiments, the processing device may iteratively perform the process of further adjusting the model parameters. When it is determined that the second iteration termination condition is satisfied, the process may proceed to 670 to terminate the iteration operation and obtain the trained image processing model. When the second iteration termination condition is not satisfied, the process may proceed back to repeat operations 630 to 650 until the second iteration termination condition is satisfied.

In some embodiments, the second iteration termination condition may be a termination condition of neural network unit training. The second iteration termination condition may include that the count of the iteration times reaches the second preset count, and/or the value of a second loss function in the iteration operation is less than or equal to a second preset loss function threshold. In some embodiments, the second loss function may include one or more differences between two groups of sample material decomposition images obtained in two adjacent iterations in the image processing model training, or two groups of updated initial material density images obtained in two adjacent iterations in the image processing model training.

In some embodiments, the second preset count may be a threshold of a total count of the iteration times of the image processing model training, for example, the threshold may be 5, 100, 500, 1000, etc. In some embodiments, when the count of the iteration times satisfies the second preset count, the image processing model training process may be terminated to obtain the trained image processing model.

In some embodiments, the second preset loss function threshold may be any reasonable threshold representing one or more differences between two groups of updated material density images obtained in two adjacent iterations. In some embodiments, the second preset loss function threshold may be expressed as any reasonable numerical value type, such as percentage (e.g., 1%, 5%, etc.), decimal (e.g., 0.01, 0.03, etc.), a fraction, an integer, etc. In some embodiments, the one or more differences between the two groups of updated material density images obtained in two adjacent iterations may include one or more differences or ratios of the image quality values of the two groups of updated material density images. In some embodiments, if the one or more differences between the two groups of updated material density images obtained in two adjacent iterations are less than or equal to the second preset loss function threshold, the image processing model training process may be terminated to obtain the trained image processing model.

In some embodiments, the second preset count and the first preset count may be different values, and the second preset loss function threshold and the first preset loss function threshold may be different values.

In 670, a trained image processing model may be obtained.

In some embodiments, the processing device may take the image processing model that satisfies the second iteration termination condition as the trained image processing model.

In some embodiments, during training of the initial neural network unit (e.g., the initial image conversion model) or the updated neural network unit (e.g., the updated image conversion model), a target loss function (e.g., the second loss function) may be optimized, and parameters of the neural network unit may be adjusted.

It should be noted that the above description of the process 600 is provided for illustrative purposes only and is not intended to limit the scope of the present disclosure. For those skilled in the art, various changes and modifications may be made according to the description of the present disclosure. However, these changes and modifications do not depart from the scope of the present disclosure. In some embodiments, the process 600 may include one or more additional operations, or the above one or more operations may be omitted. For example, a construction process of the initial neural network unit may be included before 610.

The embodiments of the present disclosure can improve the accuracy of the image processing model, improve the efficiency of the noise reduction processing and/or the artifact removal processing of the one or more material density images, and make the one or more material decomposition images obtained based on the one or more CT images or X-ray image more accurate. Because the noise reduction processing and/or the artifact removal processing using the neural network unit is repeated in an iteration operation, each iteration of the noise reduction processing and/or the artifact removal processing can improve the signal-to-noise ratio of images. After such iterative processing, the noise and/or artifact may be gradually reduced, and the effect of the noise reduction processing and/or the artifact removal processing may be improved. In the embodiments of the present disclosure, iterative decomposition may be performed while the training of the image processing model, which can make the neural network unit gradually reduce the noises of the one or more material density images in the training process, and the obtained material density images may have lower noises and higher qualities.

Having thus described the basic concepts, it may be rather apparent to those skilled in the art after reading this detailed disclosure that the foregoing detailed disclosure is intended to be presented by way of example only and is not limiting.

Meanwhile, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or characteristics may be combined as suitable in one or more embodiments of the present disclosure.

Further, it will be appreciated by one skilled in the art, aspects of the present disclosure may be illustrated and described herein in any of a number of patentable classes or context including any new and useful process, machine, manufacture, or composition of matter, or any new and useful improvement thereof. Accordingly, aspects of the present disclosure may be implemented entirely hardware, entirely software (including firmware, resident software, micro-code, etc.) or combining software and hardware implementation that may all generally be referred to herein as an "data block," "module," "engine," "unit," "component," or "system." Furthermore, aspects of the present disclosure may take the form of a computer program product embodied in one or more computer readable media having computer readable program code embodied thereon.

A computer readable signal medium may include a propagated data signal with computer readable program code embodied therein, for example, in baseband or as part of a carrier wave. Such a propagated signal may take any of a variety of forms, including electro-magnetic, optical, or the like, or any suitable combination thereof. A computer readable signal medium may be any computer readable medium that is not a computer readable storage medium and that may communicate, propagate, or transport a program for use by or in connection with an instruction execution system, apparatus, or device. Program code embodied on a computer readable signal medium may be transmitted using any appropriate medium, including wireless, wireline, optical fiber cable, RF, or the like, or any suitable combination of the foregoing.

Computer program code for carrying out operations for aspects of the present disclosure may be written in any combination of one or more programming languages, including an object oriented programming language such as Java, Scala, Smalltalk, Eiffel, JADE, Emerald, C++, C#, VB. NET, Python or the like, conventional procedural programming languages, such as the "C" programming language, Visual Basic, Fortran 2003, Perl, COBOL 2002, PHP, ABAP, dynamic programming languages such as Python, Ruby and Groovy, or other programming languages. The program code may execute entirely on the operator's computer, partly on the operator's computer, as a stand-alone software package, partly on the operator's computer and partly on a remote computer or entirely on the remote computer or server. In the latter scenario, the remote computer may be connected to the operator's computer through any type of network, including a local area network (LAN) or a wide area network (WAN), or the connection may be made to an external computer (for example, through the Internet using an Internet Service Provider) or in a cloud computing environment or offered as a service such as a Software as a Service (SaaS).

**[0140]** [0085] Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the scope of the disclosed embodiments. For example, although the implementation of various components described above may be embodied in a hardware device, it may also be implemented as a software only solution-e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. However, this disclosure does not mean that the present disclosure object requires more features than the features mentioned in the claims. Rather, claimed subject matter may lie in less than all features of a single foregoing disclosed embodiment.

In some embodiments, the numbers expressing quantities of ingredients, properties, and so forth, used to describe and claim certain embodiments of the application are to be understood as being modified in some instances by the term "about," "approximate," or "substantially." For example, "about," "approximate," or "substantially" may indicate $\pm20\%$ variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Finally, it should be understood that the embodiments described in the present disclosure merely illustrates the principles of the embodiments of the present disclosure. Other modifications may be within the scope of the present disclosure. The invention is defined by the appended claims.

# EP 4 330 835 B1

## Claims

1. A computer-implemented method, the method comprising:

   obtaining one or more initial material density images; and
   inputting the one or more initial material density images into a trained image processing model to obtain one or more target material density images;
   wherein:

   the trained image processing model includes a neural network unit and an iterative decomposition unit, the neural network unit being configured for performing the at least one of noise reduction processing or artifact removal processing on the one or more initial material density images, and the iterative decomposition unit being configured for iteratively decomposing and updating the one or more initial material density images, such that the trained image processing model simultaneously performs decomposition processing and at least one of the noise reduction processing or the artifact removal processing on the one or more initial material density images;
   for one or more iterations of the at least one of the noise reduction processing or the artifact removal processing, an input of the neural network unit in an initial iteration of the one or more iterations includes the one or more initial material density images;
   an input of the neural network unit in an m-th iteration of the one or more iterations includes one or more updated material density images determined by the iterative decomposition unit in an (m-1)th iteration of the one or more iterations, m being a positive integer greater than 1;
   for one or more iterations of the decomposition processing, an input of the iterative decomposition unit in an nth iteration of the one or more iterations includes one or more material decomposition images output by the neural network unit in the nth iteration, n being a positive integer;
   the neural network unit includes an image conversion model, and the trained image processing model is obtained according to a process including:

   updating model parameters of the initial image conversion model; and
   iteratively adjusting the model parameters of the initial image conversion model to obtain the trained image processing model;

   the updating model parameters of an initial image conversion model includes:

   obtaining a first training set, the first training set including one or more sample pairs, each of the one or more sample pairs including a sample initial material density image and a corresponding label material density image; and
   training the initial image conversion model using the first training set to update the model parameters of the initial image conversion model; and

   **characterized in that**
   the iteratively adjusting the model parameters of the initial image conversion model includes:

   inputting the one or more sample initial material density images into an updated image conversion model to obtain one or more sample material decomposition images;
   updating the one or more sample initial material density images using the iterative decomposition unit based on the one or more sample material decomposition images; and
   determining a second training set based on one or more updated sample initial material density images and one or more corresponding label material density images, and further training the updated image conversion model using the second training set to further adjust the model parameters of the initial image conversion model.

2. The method of claim 1, wherein the iterative decomposition unit includes an objective function used to obtain variate values when a sum of a data fidelity term and a data penalty term reaches a minimum value.

3. The method of claim 2, wherein the data penalty term is determined based on the neural network unit.

4. The method of any one of claims 1-3, wherein the trained image processing model is further configured to simultaneously perform the decomposition processing and the at least one of the noise reduction processing or the artifact removal processing on the one or more initial material density images using an iterative operation.

5. The method of claim 4, wherein

the one or more target material density images include material density images output by the trained image processing model when a first iteration termination condition is satisfied; and
the first iteration termination condition includes that a count of iteration times of the iterative operation reaches a first preset count, and/or a value of a first loss function in the iterative operation is less than or equal to a first preset loss function threshold.

6. The method of claim 1, wherein the first loss function includes one or more differences between two groups of material decomposition images output by the neural network unit in two adjacent iterations respectively, or one or more differences between two groups of updated material density images output by the iterative decomposition unit in two adjacent iterations.

7. The method of claim 1, wherein the iteratively adjusting the model parameters of the initial image conversion model to obtain the trained image processing model further including:
taking the one or more updated sample initial material density images as inputs of the updated image conversion model, and iteratively adjusting the model parameters of the initial image conversion model until a second iteration termination condition is satisfied to obtain the trained image processing model.

8. The method of claim 7, wherein the second iteration termination condition includes that a count of iteration times reaches a second preset count, and/or a value of a second loss function is less than or equal to a second preset loss function threshold.

9. The method of claim 8, wherein the second loss function includes one or more differences between two groups of sample material decomposition images obtained in two adjacent iterations respectively in an image processing model training process, or one or more differences between two groups of updated sample initial material density images obtained in two adjacent iterations respectively in the image processing model training process.

10. An apparatus for image processing system (100), comprising:

a scanner (110) configured to obtain one or more images to be processed; and
an image processing device (140) configured to perform operations including:

obtaining one or more initial material density images; and
inputting the one or more initial material density images into a trained image processing model to obtain one or more target material density images;
wherein:

the trained image processing model includes a neural network unit and an iterative decomposition unit, the neural network unit being configured for performing the at least one of noise reduction processing or artifact removal processing on the one or more initial material density images, and the iterative decomposition unit being configured for iteratively decomposing and updating the one or more initial material density images, such that the trained image processing model simultaneously performs decomposition processing and at least one of the noise reduction processing or the artifact removal processing on the one or more initial material density images;
for one or more iterations of the at least one of the noise reduction processing or the artifact removal processing, an input of the neural network unit in an initial iteration of the one or more iterations includes the one or more initial material density images;
an input of the neural network unit in an m-th iteration of the one or more iterations includes one or more updated material density images determined by the iterative decomposition unit in an (m-1)th iteration of the one or more iterations, m being a positive integer greater than 1;
for one or more iterations of the decomposition processing, an input of the iterative decomposition unit in an nth iteration of the one or more iterations includes one or more material decomposition images output by the neural network unit in the nth iteration, n being a positive integer;
the neural network unit includes an image conversion model, and the trained image processing model is obtained according to a process includes:

updating model parameters of the initial image conversion model; and
iteratively adjusting the model parameters of the initial image conversion model to obtain the trained image processing model;

the updating model parameters of an initial image conversion model includes:

obtaining a first training set, the first training set including one or more sample pairs, each of the one or more sample pairs including a sample initial material density image and a corresponding label material density image; and
training the initial image conversion model using the first training set to update the model parameters of the initial image conversion model; and
**characterized in that**

the iteratively adjusting the model parameters of the initial image conversion model includes:

inputting the one or more sample initial material density images into an updated image conversion model to obtain one or more sample material decomposition images;
updating the one or more sample initial material density images using the iterative decomposition unit based on the one or more sample material decomposition images; and
determining a second training set based on one or more updated sample initial material density images and one or more corresponding label material density images, and further training the updated image conversion model using the second training set to further adjust the model parameters of the initial image conversion model.

## Patentansprüche

1. Computerimplementiertes Verfahren, wobei das Verfahren Folgendes umfasst:

Erhalten eines oder mehrerer anfänglicher Materialdichtebilder; und
Eingeben des einen oder der mehreren anfänglichen Materialdichtebilder in ein trainiertes Bildverarbeitungsmodell, um ein oder mehrere Materialdichte-Zielbilder zu erhalten;
wobei:

das trainierte Bildverarbeitungsmodell eine neuronale Netzwerkeinheit und eine iterative Zerlegungseinheit einschließt, wobei die neuronale Netzwerkeinheit zum Durchführen des mindestens einen einer Rauschunterdrückungsverarbeitung oder Artefaktentfernungsverarbeitung an dem einen oder den mehreren anfänglichen Materialdichtebildern konfiguriert ist und die iterative Zerlegungseinheit zum iterativen Zerlegen und Aktualisieren des einen oder der mehreren anfänglichen Materialdichtebilder konfiguriert ist, sodass das trainierte Bildverarbeitungsmodell gleichzeitig Zerlegungsverarbeitung und mindestens eines der Rauschunterdrückungsverarbeitung oder Artefaktentfernungsverarbeitung an dem einen oder den mehreren anfänglichen Materialdichtebildern durchführt;
für eine oder mehrere Iterationen des mindestens einen der Rauschunterdrückungsverarbeitung oder der Artefaktentfernungsverarbeitung eine Eingabe der neuronalen Netzwerkeinheit in eine anfängliche Iteration der einen oder mehreren Iterationen das eine oder die mehreren anfänglichen Materialdichtebilder einschließt;
eine Eingabe der neuronalen Netzwerkeinheit in eine $m$-te Iteration der einen oder mehreren Iterationen ein oder mehrere aktualisierte Materialdichtebilder einschließt, die durch die iterative Zerlegungseinheit in einer ($m$-1)-ten Iteration der einen oder mehreren Iterationen bestimmt wurden, wobei $m$ eine positive ganze Zahl größer als 1 ist;
für eine oder mehrere Iterationen der Zerlegungsverarbeitung eine Eingabe der iterativen Zerlegungseinheit in eine $n$-te Iteration der einen oder mehreren Iterationen ein oder mehrere Materialzerlegungsbilder einschließt, die durch die neuronale Netzwerkeinheit in der $n$-ten Iteration ausgegeben werden, *wobei n* eine positive ganze Zahl ist;
die neuronale Netzwerkeinheit ein Bildkonvertierungsmodell einschließt und das trainierte Bildverarbeitungsmodell gemäß einem Prozess erhalten wird, der Folgendes einschließt:

Aktualisieren von Modellparametern des anfänglichen Bildkonvertierungsmodells; und

iteratives Anpassen der Modellparameter des anfänglichen Bildkonvertierungsmodells, um das trainierte Bildverarbeitungsmodell zu erhalten;

wobei das Aktualisieren von Modellparametern eines anfänglichen Bildkonvertierungsmodells Folgendes einschließt:

Erhalten eines ersten Trainingssatzes, wobei der erste Trainingssatz ein oder mehrere Probenpaare einschließt, wobei jeder des einen oder der mehreren Probenpaare ein anfängliche Materialdichte-Probenbild und ein entsprechendes Etikettenmaterialdichtebild einschließt; und

Trainieren des anfänglichen Bildkonvertierungsmodells unter Verwendung des ersten Trainingssatzes, um die Modellparameter des anfänglichen Bildkonvertierungsmodells zu aktualisieren; und

**dadurch gekennzeichnet, dass**

das iterative Anpassen der Modellparameter des anfänglichen Bildkonvertierungsmodus Folgendes einschließt:

Eingeben des einen oder der mehreren anfänglichen Materialdichte-Probenbilder in ein aktualisiertes Bildkonvertierungsmodell, um ein oder mehrere Materialzerlegungs-Probenbilder zu erhalten;

Aktualisieren des einen oder der mehreren anfänglichen Materialdichte-Probenbilder unter Verwendung der iterativen Zerlegungseinheit basierend auf dem einen oder den mehreren Materialzerlegung-Probenbildern; und

Bestimmen eines zweiten Trainingssatzes basierend auf einem oder mehreren aktualisierten anfänglichen Materialdichte-Probenbildern und einem oder mehreren entsprechenden Etikettenmaterialdichtebildern und weiteres Trainieren des aktualisierten Bildkonvertierungsmodells unter Verwendung des zweiten Trainingssatzes, um die Modellparameter des anfänglichen Bildkonvertierungsmodells weiter anzupassen.

2. Verfahren nach Anspruch 1, wobei die iterative Zerlegungseinheit eine Zielfunktion einschließt, die verwendet wird, um Variablenwerte zu erhalten, wenn eine Summe eines Datentreueterms und eines Datenstrafterms einen Mindestwert erreicht.

3. Verfahren nach Anspruch 2, wobei der Datenstrafterm basierend auf der neuronalen Netzwerkeinheit bestimmt wird.

4. Verfahren nach einem der Ansprüche 1-3, wobei das trainierte Bildverarbeitungsmodell weiter so konfiguriert ist, dass es die Zerlegungsverarbeitung und mindestens eines von der Rauschunterdrückungsverarbeitung oder Artefaktentfernung gleichzeitig an dem einen oder den mehreren anfänglichen Materialdichtebildern unter Verwendung einer iterativen Operation durchführt.

5. Verfahren nach Anspruch 4, wobei

das eine oder die mehreren Materialdichte-Zielbilder Materialdichtebilder einschließen, die durch das trainierte Bildverarbeitungsmodell ausgegeben werden, wenn eine erste Iterationsbeendigungsbedingung erfüllt ist; und

die erste Iterationsbeendigungsbedingung einschließt, dass eine Anzahl von Iterationen der iterativen Operation eine erste voreingestellte Anzahl erreicht und/oder ein Wert einer ersten Verlustfunktion in der iterativen Operation kleiner oder gleich groß wie ein erster voreingestellter Schwellenwert der Verlustfunktion ist.

6. Verfahren nach Anspruch 1, wobei die erste Verlustfunktion einen oder mehrere Unterschiede zwischen zwei Gruppen von Materialzerlegungsbildern einschließt, die durch die neuronale Netzwerkeinheit in jeweils zwei benachbarten Iterationen ausgegeben werden, oder einen oder mehrere Unterschiede zwischen zwei Gruppen von aktualisierten Materialdichtebildern, durch die iterative Zerlegungseinheit in zwei benachbarten Iterationen ausgegeben werden.

7. Verfahren nach Anspruch 1, wobei das iterative Anpassen der Modellparameter des anfänglichen Bildkonvertierungsmodells zum Erhalten des trainierten Bildverarbeitungsmodells weiter Folgendes einschließt:

Hernehmen der einen oder mehreren aktualisierten anfänglichen Materialdichte-Probenbilder als Eingaben des aktualisierten Bildkonvertierungsmodells und iteratives Anpassen der Modellparameter des anfänglichen Bildkonvertierungsmodells, bis eine zweite Iterationsbeendigungsbedingung erfüllt ist, um das trainierte Bildverarbeitungsmodell zu erhalten.

8. Verfahren nach Anspruch 7, wobei die zweite Iterationsbeendigungsbedingung einschließt, dass eine Anzahl von Iterationen eine zweite voreingestellte Anzahl erreicht und/oder ein Wert einer zweiten Verlustfunktion kleiner oder gleich groß wie ein zweiter voreingestellter Verlustfunktionsschwellenwert ist.

9. Verfahren nach Anspruch 8, wobei die zweite Verlustfunktion einen oder mehrere Unterschiede zwischen zwei Gruppen von Materialzerlegungs-Probenbildern einschließt, die jeweils in zwei benachbarten Iterationen in einem Trainingsprozess für ein Bildverarbeitungsmodell erhalten wurden, oder einen oder mehrere Unterschiede zwischen zwei Gruppen von aktualisierten anfänglichen Materialdichte-Probenbildern, die jeweils in zwei benachbarten Iterationen im Trainingsprozess für ein Bildverarbeitungsmodell erhalten wurden.

10. Einrichtung für ein Bildverarbeitungssystem (100), umfassend:

einen Scanner (110), der so konfiguriert ist, dass er ein oder mehrere zu verarbeitende Bilder erhält; und
eine Bildverarbeitungsvorrichtung (140), die so konfiguriert ist, dass sie Operationen durchführt, die Folgendes einschließen:

Erhalten eines oder mehrerer anfänglicher Materialdichtebilder; und
Eingeben des einen oder der mehreren anfänglichen Materialdichtebilder in ein trainiertes Bildverarbeitungsmodell, um ein oder mehrere Materialdichte-Zielbilder zu erhalten;
wobei:

das trainierte Bildverarbeitungsmodell eine neuronale Netzwerkeinheit und eine iterative Zerlegungseinheit einschließt, wobei die neuronale Netzwerkeinheit zum Durchführen des mindestens einen einer Rauschunterdrückungsverarbeitung oder Artefaktentfernungsverarbeitung an dem einen oder den mehreren anfänglichen Materialdichtebildern konfiguriert ist und die iterative Zerlegungseinheit zum iterativen Zerlegen und Aktualisieren des einen oder der mehreren anfänglichen Materialdichtebilder konfiguriert ist, sodass das trainierte Bildverarbeitungsmodell gleichzeitig Zerlegungsverarbeitung und mindestens eines der Rauschunterdrückungsverarbeitung oder Artefaktentfernungsverarbeitung an dem einen oder den mehreren anfänglichen Materialdichtebildern durchführt;
für eine oder mehrere Iterationen des mindestens einen der Rauschunterdrückungsverarbeitung oder der Artefaktentfernungsverarbeitung eine Eingabe der neuronalen Netzwerkeinheit in eine anfängliche Iteration der einen oder mehreren Iterationen das eine oder die mehreren anfänglichen Materialdichtebilder einschließt;
eine Eingabe der neuronalen Netzwerkeinheit in eine $m$-te Iteration der einen oder mehreren Iterationen ein oder mehrere aktualisierte Materialdichtebilder einschließt, die durch die iterative Zerlegungseinheit in einer ($m$-1)-ten Iteration der einen oder mehreren Iterationen bestimmt wurden, wobei $m$ eine positive ganze Zahl größer als 1 ist;
für eine oder mehrere Iterationen der Zerlegungsverarbeitung eine Eingabe der iterativen Zerlegungseinheit in eine $n$-te Iteration der einen oder mehreren Iterationen ein oder mehrere Materialzerlegungsbilder einschließt, die durch die neuronale Netzwerkeinheit in der $n$-ten Iteration ausgegeben werden, *wobei n* eine positive ganze Zahl ist;
die neuronale Netzwerkeinheit ein Bildkonvertierungsmodell einschließt und das trainierte Bildverarbeitungsmodell gemäß einem Prozess erhalten wird, der Folgendes einschließt:

Aktualisieren von Modellparametern des anfänglichen Bildkonvertierungsmodells; und
iteratives Anpassen der Modellparameter des anfänglichen Bildkonvertierungsmodells, um das trainierte Bildverarbeitungsmodell zu erhalten;
wobei das Aktualisieren von Modellparametern eines anfänglichen Bildkonvertierungsmodells Folgendes einschließt:

Erhalten eines ersten Trainingssatzes, wobei der erste Trainingssatz ein oder mehrere Probenpaare einschließt, wobei jeder des einen oder der mehreren Probenpaare ein anfängliche Materialdichte-Probenbild und ein entsprechendes Etikettenmaterialdichtebild einschließt; und
Trainieren des anfänglichen Bildkonvertierungsmodells unter Verwendung des ersten Trainingssatzes, um die Modellparameter des anfänglichen Bildkonvertierungsmodells zu aktua-

**EP 4 330 835 B1**

lisieren; und

**dadurch gekennzeichnet, dass**

das iterative Anpassen der Modellparameter des anfänglichen Bildkonvertierungsmodus Folgendes einschließt:

Eingeben des einen oder der mehreren anfänglichen Materialdichte-Probenbilder in ein aktualisiertes Bildkonvertierungsmodell, um ein oder mehrere Materialzerlegungs-Probenbilder zu erhalten;

Aktualisieren des einen oder der mehreren anfänglichen Materialdichte-Probenbilder unter Verwendung der iterativen Zerlegungseinheit basierend auf dem einen oder den mehreren Materialzerlegung-Probenbildern; und

Bestimmen eines zweiten Trainingssatzes basierend auf einem oder mehreren aktualisierten anfänglichen Materialdichte-Probenbildern und einem oder mehreren entsprechenden Etikettenmaterialdichtebildern und weiteres Trainieren des aktualisierten Bildkonvertierungsmodells unter Verwendung des zweiten Trainingssatzes, um die Modellparameter des anfänglichen Bildkonvertierungsmodells weiter anzupassen.

**Revendications**

1. Procédé mis en œuvre par ordinateur, le procédé comprenant :

l'obtention d'une ou plusieurs images initiales de densité de matériau ; et

l'entrée d'une ou plusieurs images de densité de matériau initiales dans un modèle de traitement d'image entraîné pour obtenir une ou plusieurs images de densité de matériau cibles ;

dans lequel :

le modèle de traitement d'image entraîné inclut une unité de réseau neuronal et une unité de décomposition itérative, l'unité de réseau neuronal étant configurée pour mettre en œuvre au moins un traitement de réduction de bruit ou un traitement de suppression d'artefact sur l'une ou les plusieurs images de densité de matériau initiales, et l'unité de décomposition itérative étant configurée pour décomposer et mettre à jour de manière itérative l'une ou les plusieurs images de densité de matériau initiales, de sorte que le modèle de traitement d'image entraîné met en œuvre simultanément un traitement de décomposition et au moins un traitement de réduction de bruit ou un traitement de suppression d'artefact sur l'une ou les plusieurs images de densité de matériau initiales ;

pour une ou plusieurs itérations du au moins un traitement de réduction de bruit ou de traitement de suppression d'artefact, une entrée de l'unité de réseau neuronal dans une itération initiale de l'une ou des plusieurs itérations inclut l'une ou les plusieurs images de densité de matériau initiales ;

une entrée de l'unité de réseau neuronal dans une $m$e itération de l'une ou des plusieurs des itérations inclut une ou plusieurs images de densité de matériau mises à jour déterminées par l'unité de décomposition itérative dans une ($m$-1)e itération de l'une ou des plusieurs itérations, $m$ étant un entier positif supérieur à 1 ;

pour une ou plusieurs itérations du traitement de décomposition, une entrée de l'unité de décomposition itérative dans une $n$e itération de l'une ou des plusieurs itérations inclut une ou plusieurs images de décomposition de matériau émises par l'unité de réseau neuronal dans la $n$e itération, $n$ étant un entier positif;

l'unité de réseau neuronal inclut un modèle de conversion d'image, et le modèle de traitement d'image entraîné est obtenu selon un processus incluant :

la mise à jour des paramètres de modèle du modèle de conversion d'image initial ; et

l'ajustement de manière itérative les paramètres de modèle du modèle de conversion d'image initial pour obtenir le modèle de traitement d'image entraîné ;

la mise à jour de paramètres de modèle d'un modèle de conversion d'image initial inclut :

l'obtention d'un premier ensemble d'entraînement, le premier ensemble d'entraînement incluant une ou plusieurs paires d'échantillons, chacune de l'une ou des plusieurs paires d'échantillons incluant une image de densité de matériau initiale d'échantillon et une image de densité de matériau d'étiquette correspondante ; et

l'entraînement du modèle de conversion d'image initial en utilisant le premier ensemble d'entraînement pour mettre à jour les paramètres de modèle du modèle de conversion d'image initial ; et

**caractérisée en ce que**
l'ajustement itératif des paramètres de modèle du mode de conversion d'image initial inclut :

l'entrée d'une ou plusieurs images de densité de matériau initiales d'échantillon dans un modèle de conversion d'image mis à jour pour obtenir une ou plusieurs images de décomposition de matériau d'échantillon ;
la mise à jour de l'une ou les plusieurs images de densité de matériau initiales d'échantillon en utilisant l'unité de décomposition itérative sur la base d'une ou de plusieurs images de décomposition de matériau d'échantillon ; et
la détermination d'un second ensemble d'entraînement sur la base d'une ou de plusieurs images de densité de matériau initiales d'échantillon mises à jour et d'une ou de plusieurs images de densité de matériau d'étiquette correspondantes, et entraîner en outre le modèle de conversion d'image mis à jour en utilisant le second ensemble d'entraînement pour ajuster en outre les paramètres de modèle du modèle de conversion d'image initiale.

**2.** Procédé selon la revendication 1, dans lequel l'unité de décomposition itérative inclut une fonction objective utilisée pour obtenir des valeurs variables lorsqu'une somme d'un terme de fidélité de données et d'un terme de pénalité de données atteint une valeur minimale.

**3.** Procédé selon la revendication 2, dans lequel le terme de pénalité de données est déterminé sur la base de l'unité de réseau neuronal.

**4.** Procédé selon l'une quelconque des revendications 1-3, dans lequel le modèle de traitement d'image entraîné est en outre configuré pour mettre en œuvre simultanément le traitement de décomposition et l'au moins un traitement de réduction de bruit ou le traitement de suppression d'artefact sur l'une ou les plusieurs images de densité de matériau initiales en utilisant une opération itérative.

**5.** Procédé selon la revendication 4, dans lequel

l'une ou les plusieurs images de densité de matériau cibles incluent des images de densité de matériau émises par le modèle de traitement d'image entraîné lorsqu'une première condition de fin d'itération est satisfaite ; et
la première condition de fin d'itération inclut qu'un nombre de fois d'itération de l'opération itérative atteint un premier nombre prédéfini, et/ou une valeur d'une première fonction de perte dans l'opération itérative est inférieure ou égale à un premier seuil de fonction de perte prédéfini.

**6.** Procédé selon la revendication 1, dans lequel la première fonction de perte inclut une ou plusieurs différences entre deux groupes d'images de décomposition de matériau émises par l'unité de réseau neuronal dans deux itérations adjacentes respectivement, ou une ou plusieurs différences entre deux groupes d'images de densité de matériau mises à jour émises par l'unité de décomposition itérative dans deux itérations adjacentes.

**7.** Procédé selon la revendication 1, dans lequel l'ajustement itératif des paramètres de modèle du modèle de conversion d'image initial pour obtenir le modèle de traitement d'image entraîné incluant en outre :
la prise en compte de l'une ou les plusieurs images de densité de matériau initiales d'échantillon mises à jour comme entrées du modèle de conversion d'image mis à jour, et ajuster de manière itérative les paramètres de modèle du modèle de conversion d'image initial jusqu'à ce qu'une seconde condition de fin d'itération soit satisfaite pour obtenir le modèle de traitement d'image entraîné.

**8.** Procédé selon la revendication 7, dans lequel la seconde condition de fin d'itération inclut le fait qu'un nombre de fois d'itération atteint un second nombre prédéfini, et/ou une valeur d'une seconde fonction de perte est inférieure ou égale à un second seuil de fonction de perte prédéfini.

**9.** Procédé selon la revendication 8, dans lequel la seconde fonction de perte inclut une ou plusieurs différences entre deux groupes d'images de décomposition de matériau d'échantillon obtenues dans deux itérations adjacentes respectivement dans un processus d'entraînement de modèle de traitement d'image, ou une ou plusieurs différences entre deux groupes d'images de densité de matériau initiale d'échantillon mises à jour obtenues dans deux itérations adjacentes respectivement dans le processus d'entraînement de modèle de traitement d'image.

**10.** Appareil pour système de traitement d'image (100), comprenant :

un scanner (110) configuré pour obtenir une ou plusieurs images à traiter ; et
un dispositif de traitement d'image (140) configuré pour mettre en œuvre des opérations incluant :

l'obtention d'une ou plusieurs images initiales de densité de matériau ; et
l'entrée d'une ou plusieurs images de densité de matériau initiales dans un modèle de traitement d'image entraîné pour obtenir une ou plusieurs images de densité de matériau cibles ;
dans lequel :

le modèle de traitement d'image entraîné inclut une unité de réseau neuronal et une unité de décomposition itérative, l'unité de réseau neuronal étant configurée pour mettre en œuvre au moins un traitement de réduction de bruit ou un traitement de suppression d'artefact sur l'une ou les plusieurs images de densité de matériau initiales, et l'unité de décomposition itérative étant configurée pour décomposer et mettre à jour de manière itérative l'une ou les plusieurs images de densité de matériau initiales, de sorte que le modèle de traitement d'image entraîné met en œuvre simultanément un traitement de décomposition et au moins un traitement de réduction de bruit ou un traitement de suppression d'artefact sur l'une ou les plusieurs images de densité de matériau initiales ;
pour une ou plusieurs itérations du au moins un traitement de réduction de bruit ou de traitement de suppression d'artefact, une entrée de l'unité de réseau neuronal dans une itération initiale de l'une ou des plusieurs itérations inclut l'une ou les plusieurs images de densité de matériau initiales ;
une entrée de l'unité de réseau neuronal dans une me itération de l'une ou des plusieurs des itérations inclut une ou plusieurs images de densité de matériau mises à jour déterminées par l'unité de décomposition itérative dans une ($m$-1)e itération de l'une ou des plusieurs itérations, $m$ étant un entier positif supérieur à 1 ;
pour une ou plusieurs itérations du traitement de décomposition, une entrée de l'unité de décomposition itérative dans une ne itération de l'une ou des plusieurs itérations inclut une ou plusieurs images de décomposition de matériau émises par l'unité de réseau neuronal dans la ne itération, $n$ étant un entier positif;
l'unité de réseau neuronal inclut un modèle de conversion d'image, et le modèle de traitement d'image entraîné est obtenu selon un processus inclut :

la mise à jour des paramètres de modèle du modèle de conversion d'image initial ; et
l'ajustement de manière itérative les paramètres de modèle du modèle de conversion d'image initial pour obtenir le modèle de traitement d'image entraîné ;
la mise à jour de paramètres de modèle d'un modèle de conversion d'image initial inclut :

l'obtention d'un premier ensemble d'entraînement, le premier ensemble d'entraînement incluant une ou plusieurs paires d'échantillons, chacune de l'une ou des plusieurs paires d'échantillons incluant une image de densité de matériau initiale d'échantillon et une image de densité de matériau d'étiquette correspondante ; et
l'entraînement du modèle de conversion d'image initial en utilisant le premier ensemble d'entraînement pour mettre à jour les paramètres de modèle du modèle de conversion d'image initial ; et
**caractérisée en ce que**
l'ajustement itératif des paramètres de modèle du mode de conversion d'image initial inclut :

l'entrée d'une ou plusieurs images de densité de matériau initiales d'échantillon dans un modèle de conversion d'image mis à jour pour obtenir une ou plusieurs images de décomposition de matériau d'échantillon ;
la mise à jour de l'une ou les plusieurs images de densité de matériau initiales d'échantillon en utilisant l'unité de décomposition itérative sur la base d'une ou de plusieurs images de décomposition de matériau d'échantillon ; et
la détermination d'un second ensemble d'entraînement sur la base d'une ou de plusieurs images de densité de matériau initiales d'échantillon mises à jour et d'une ou de plusieurs images de densité de matériau d'étiquette correspondantes, et entraîner en outre le modèle de conversion d'image mis à jour en utilisant le second ensemble d'entraînement pour ajuster en outre les paramètres de modèle du modèle de conversion d'image initiale.

<u>100</u>

**FIG. 1**

**200**

**FIG. 2**

**FIG. 3**

**400**

Obtaining one or more images to be processed  410

Determining one or more initial material density images based on the one or more images to be processed  420

Inputting the one or more initial material density images into the trained image processing model to obtain one or more target material density images  430

**FIG. 4**

**500**

Determining one or more material decomposition images based on the one or more initial material density images or one or more updated material density images through the neural network unit ~510

↓

Determining one or more updated material density images using the iterative decomposition unit based on the one or more material decomposition images ~520

↓

Whether the first iteration termination condition is satisfied? ~530

No →

Yes ↓

Determining the one or more target material density images ~540

FIG. 5

**600**

Obtaining a first training set $\qquad$ 610

Training an initial image conversion model using the first training set to update model parameters of the initial image conversion model $\qquad$ 620

Inputting one or more sample initial material density images into an updated image conversion model to obtain one or more sample material decomposition images $\qquad$ 630

Updating the one or more sample initial material density images using an iterative decomposition unit based on the one or more sample material decomposition images $\qquad$ 640

Determining a second training set based on the one or more updated sample initial material density images, and further training the updated image conversion model using the second training set $\qquad$ 650

No — Whether the second iteration termination condition is satisfied? $\qquad$ 660

Yes

Obtaining the trained image processing model $\qquad$ 670

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202111070115

**Non-patent literature cited in the description**

- An Improved Iterative Neural Network for High-Quality Image-Domain Material Decomposition in Dual-Energy CT. **ZHIPENG LI ET**. ARXIV.ORG. CORNELL UNIVERSITY LIBRARY, 02 December 2020

- Image-Domain Material Decomposition Using an Iterative Neural Network for Dual-Energy CT. **LI ZHIPENG et al.** 2020 IEEE 17TH INTERNATIONAL SYMPOSIUM ON BIOMEDICAL IMAGING (ISBI). IEEE, 03 April 2020, 651-655